(19) Europäisches Patentamt
European Patent Office
Office européen des brevets

(11) EP 1 897 941 A1

# (12) EUROPEAN PATENT APPLICATION

(43) Date of publication:
**12.03.2008 Bulletin 2008/11**

(21) Application number: **06018993.3**

(22) Date of filing: **11.09.2006**

(51) Int Cl.:
***C12N 15/11*** *(2006.01)* ***A61K 31/713*** *(2006.01)*
*A61P 35/00* *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicants:
- **MAX-DELBRÜCK-CENTRUM FÜR MOLEKULARE MEDIZIN**
  **13125 Berlin (DE)**
- **Charité-Universitätsmedizin Berlin**
  **10117 Berlin (DE)**

(72) Inventors:
- **Kemmner, Wolfgang**
  **13469 Berlin (DE)**
- **Moesta, Thomas**
  **13469 Berlin (DE)**
- **Schlag, Peter-Michael**
  **13467 Berlin (DE)**

(74) Representative: **Bohmann, Armin K.**
**Bohmann & Loosen**
**Anwaltssozietät**
**Nymphenburger Strasse 1**
**80335 München (DE)**

## (54) Means for increasing intracellular levels of protoporphyrin IX and use thereof

(57) The present invention is related to a nucleic acid molecule comprising a double-stranded structure, whereby the double-stranded structure comprises a first strand and a second strand, whereby the first strand comprises a first stretch of contiguous nucleotides and the first stretch is at least partially complementary to a target nucleic acid, and whereby the second strand comprises a second stretch of contiguous nucleotides and whereby said second stretch is at least partially complementary to the first stretch, whereby the first stretch comprises a nucleic acid sequence which is complementary to a nucleotide sequence starting from position 383, 1013 or 1982 of the nucleic acid according to SEQ.ID.No. 1.

EP 1 897 941 A1

## Description

The present invention is related to a nucleic acid molecule comprising a double-stranded structure, a vector coding for such nucleic acid, a composition, preferably a pharmaceutical composition comprising such composition and the use of such nucleic acid for the manufacture of a medicament.

Protoporphyrin IX which is also referred to as PpIX, is the fluorescent heme precursor which has been shown useful for the detection and treatment of neoplastic diseases (Barr H. et al., Lancet, 348: 584-585, 1996, Koenig, F. et al., Science, 292: 1401-1403, 2001, and Gossner, L. et al., Cancer, 86: 1921-1928, 1999). Such treatment is based on intracellular PpIX accumulation which is the basis for a photochemotherapteutic method for the treatment of this kind of diseases.

Heme containing enzymes are essential for cellular energy metabolism. In the heme synthesis pathway, 5-aminolevulinic acid (ALA) is formed from glycine and succinyl coenzyme A (CoA) by aminolevulinic acid synthase (ALA-S). Subsequently, porphobilinogen deaminase (PBG-D) katalyses the formation of uroporphyrinogen from porphobilinogen. The last step is the incorporation of iron into protoporphyrin IX (PpIX), which takes place in the mitochondria. This latter process is controlled by human ferrochelatase (EC 4.99.1.1), which is localised within the inner mitochondrial membrane.

PpIX based photochemotherapeutic methods were thus focusing on the specific increase of the intracellular content of PpIX in neoplastic tissues.

Given the fact that the rate-limiting step of heme synthesis is the conversion of glycine and succinyl CoA to aminolevulinic acid (ALA) which is under negative feedback control by heme, the exogenous addition of 5' aminolevulinic acid (ALA) is one of the options at hand to increase PpIX accumulation in neoplastic tissue.

The problem underlying the present invention is thus to provide a means for increasing intracellular accumulation of PpIX in neoplastic tissue. A further problem underlying the present invention is to provide medicaments which are suitable for diagnosis and the treatment of neoplastic diseases inflammatory and other systemic diseases.

The problem underlying the present invention is solved in a first aspect by a nucleic acid molecule comprising a double-stranded structure, whereby the double-stranded structure comprises a first strand and a second strand, whereby the first strand comprises a first stretch of contiguous nucleotides and the first stretch is at least partially complementary to a target nucleic acid, and whereby the second strand comprises a second stretch of contiguous nucleotides and whereby said second stretch is at least partially complementary to the first stretch, whereby the first stretch comprises a nucleic acid sequence which is complementary to a nucleotide sequence starting from position 383, 1013 or 1982 of the nucleic acid according to SEQ.ID.No. 1.

In an embodiment of the first aspect the first stretch and/or the second stretch comprises from 18 to 29 consecutive nucleotides, preferably 19 to 25 consecutive nucleotides and more preferably 19 to 23 consecutive nucleotides.

In a further embodiment of the first aspect the first strand consists of the first stretch and/or the second strand consists of the second stretch.

In a preferred embodiment

- the first stretch consists of a nucleotide sequence according to SEQ.ID.No.2 and/or whereby the second stretch consists of a nucleotide sequence according to SEQ.ID.No.3,
- the first stretch consists of a nucleotide sequence according to SEQ.ID.No.4 and/or whereby the second stretch consists of a nucleotide sequence according to SEQ.ID.No.5,
- the first stretch consists of a nucleotide sequence according to SEQ.ID.No.6 and/or whereby the second stretch consists of a nucleotide sequence according to SEQ.ID.No.7.

In an embodiment of the first aspect the first stretch comprises a plurality of group of modified nucleotides having a modification at the 2' position, whereby within the stretch each group of modified nucleotides is flanked on one or both sides by a flanking group of nucleotides, whereby the flanking nucleotides forming the flanking group of nucleotides is either an unmodified nucleotide or a nucleotide having a modification different from the modification of the modified nucleotides.

In a further embodiment the preferred nucleic acid molecule is as follows:

5' gauucaagagcaguaccgc 3' (second strand and second stretch, respectively)

3' cuaaguucucgucauggcg 5' (first strand and first stretch, respectively);

5' gaauauccucuugguuccg 3' (second strand and second stretch, respectively)

3' cuuauaggagaaccaaggc 5' (first strand and first stretch, respectively); or

5' guacaguguucaugauacg 3' (second strand and second stretch, respectively)

3' caugucacaaguacuaugc 5' (first strand and first stretch, respectively).

In a further embodiment of the first aspect the first stretch and/or the second stretch comprises a pattern of groups of modified nucleotides and/or a pattern of flanking groups of nucleotides.

In a further embodiment of the first aspect the first stretch and/or the second stretch comprise at the 3' end a dinucleotide, whereby such dinucleotide is preferably TT.

In a further embodiment of the first aspect the the length of the first stretch and/or of the second stretch consists of either 19 or 21 nucleotides.

In a further embodiment of the first aspect the the first and/or the second stretch comprise an overhang of 1 to 5 nucleotides at the 3' end.

In a further embodiment of the first aspect the the length of the double-stranded structure is from about 16 to 24 nucleotide pairs, preferably 20 to 22 nucleotide pairs.

In a further embodiment of the first aspect the first strand and the second strand are covalently linked to each other, preferably the 3' end of the first strand is covalently linked to the 5' end of the second strand.

The problem underlying the present invention is solved in a second aspect by a vector, preferably an expression vector, comprising or coding for a nucleic acid according to the first aspect..

The problem underlying the present invention is solved in a third aspect by a composition, preferably a pharmaceutical composition, comprising a nucleic acid according to the first aspect or a vector according to the second aspect.

In a preferred embodiment the composition is a pharmaceutical composition for the treatment of a disease, whereby the disease is preferably selected from the group comprising neoplastic diseases, inflammatory and other systemic diseases.

The problem underlying the present invention is solved in a fourth aspect by the use of a nucleic acid according to the first aspect for the manufacture of a medicament, whereby such medicament is preferably used together with photodynamic treatment, radiotherapy, hyperthermia or chemotherapy.

The problem underlying the present invention is solved in a fifth aspect by the use of a nucleic acid according to the first aspect for the manufacture of a diagnostic, preferably a diagnostic for any of the diseases described herein, preferably any of the diseases for the treatment and/or prevention of which the nucleic acid molecule according to the present invention is used.

The problem underlying the present invention is solved in a sixth aspect by the use of means for the detection of increased PpIX formation as known to the ones skilled in the art, for determining whether a patient can be treated using the nucleic acid molecule of the present invention. Preferably such determination of an increased PpIX formation is performed on a patient or a sample of such patient who is suspected of suffering or being at risk of suffering from any of the diseases described herein, preferably any of the diseases for the treatment and/or prevention of which the nucleic acid molecule according to the present invention is used.

The present inventors have surprisingly found that rather than increasing the rate-limiting step of heme synthesis, i. e. increasing conversion of glycine and succinyl CoA to aminolevulinic acid (ALA) or by-passing such rate-limiting step by adding exogenous 5'aminolevalinic acid, down-regulating the expression of ferrochelatase (FC) is a suitable means for increasing intracellular accumulation of PpIX and thus to obtain an enhanced PpIX fluorescence suitable in photochemotherapeutic approaches.

By addressing the ferrochelatase downregulation at the mRNA level using the nucleic acids according to the present invention, the present inventors have departed from the approach pursued in the prior art, namely to compensate the rate limiting step for the synthesis of PpIX. Additionally, by doing so the present inventors have departed from the controversially discussed option to modify the activity level of ferrochelatase for increasing intracellular levels of PpIX. Among others, it was discussed in the prior art that the selective accumulation of PpIX is caused by a relative ferrochelatase activity deficiency in malignant tissue (van Helegersberg, R. et al., Gastroenterology, 103: 647-651, 1992). However, others like Hua et al. (Hua, Z., Cancer Res, 55: 1723-1731, 1995) did not find any direct relationship between the level of activities of the enzymes involved in heme synthesis and the amount of PpIX formed. A different approach was suggested by Hinnen, P. et al., BR. J. Cancer, 78: 679-682, 1998) who suggested that PpIX would accumulate because of an imbalance between PBG-D and ferrochelatase activities. Still others like Taketani et al. (Taketani, S. et al., Eur. J. Biochem., 267 : 4685-4692, 2000) found that intracellular iron levels contributed to the regulation of heme biosynthesis by controlling ferrochelatase activity. A low ferrochelatase activity at the enzyme level was also reported by Dailey et al. (Dailey, H. A., Biochem. J., 223: 441-445, 1984).

In view of this highly controversial and obviously unsatisfying efforts to explain increased levels of PpIX and to derive there from a technical teaching for photochemotherapeutic approaches in the treatment of neoplastic diseases, the one skilled in the art was not prompted to assume that in the effort to increase the intracellular content of PpIX in neoplastic tissues, the knockdown of mRNA encoding for ferrochelatase would be a suitable approach for the treatment of neoplastic diseases and, respectively, providing the conditions suitable for the treatment of such neoplastic tissues, preferably by photochemotherapeutic approaches.

As shown in more detail in the example part, the present inventor could show that actually the downregulation of mRNA expression of ferrochelatase is a suitable means to increase intracellular PpIX concentration. Insofar, any functional nucleic acid having this effect is thus suitable and can in accordance with the present invention be used for the treatment of such kind of disease and for increasing the intracellular PpIX content, respectively, providing the basis for the treatment of this kind of diseases, preferably by photochemotherapeutic approaches.

A particularly preferred form of this kind of functional nucleic acids are siRNA molecules, antisense molecules and ribozymes as described herein in more detail.

The diseases which can thus be treated are those which involve, either directly or indirectly, one or several neoplastic tissues. Insofar, and in an embodiment, the respective diseases are neoplastic diseases. A further group of diseases which can thus be treated are inflammatory diseases. It will be acknowledged by the ones skilled in the art that using the nucleic acid molecules according to the present invention or any other means described herein for therapeutic use, also other systemic diseases may treated and/or diagnosed. The reason for this use resides in the omnipresence of the heme synthetic pathway providing for protoporphyrin-IX playing thus also a role in systemic diseases that involve many organs or the whole body. Such other systemic diseases comprise, but are not limited to, diabetes mellitus, an imbalance in blood glucose (sugar) levels; acquired immunodeficiency syndrome (AIDS), a life-threatening disease caused by a virus that cripples the body's immune defenses; Graves' disease - a thyroid disorder, most often in women, which can cause a goiter (swelling in the front part of the neck) and protruding eyes; sarcoidosis - a disease that mainly affects the lungs, brain, joints and eyes, found most often in young African-American women; systemic lupus erythematosus - a connective tissue disorder involving mainly the skin, joints and kidneys; rheumatoid arthritis; hypertension (high blood pressure); atherosclerosis, more preferably hardening of the arteries; sickle cell disease - an inherited blood disorder that can block circulation throughout the body, primarily affecting African-Americans; and multiple sclerosis - a disease that damages nerve coverings, causing weakness, coordination and speech disturbances.

As preferably used herein, the term neoplastic tissues refers to tissues which are generated by an organism, tissue or cells of such organism which are not intended to be generated and which are deemed as pathologic, i. e. not present in a subject not suffering from such a respective disease. Also, as preferably used herein, a neoplastic disease is any disease which, either directly or indirectly, arises from the presence of a neoplastic tissue, whereby preferably such neoplastic tissue rises from the dysregulated or uncontrolled, preferably autonomous growth of a/the tissue. The term neoplastic diseases preferably also comprises benign as well as malignant neoplastic diseases. More preferably, the neoplastic diseases are selected from the group comprising diseases which can, in accordance with international classification of diseases such as maybe taken from **http://www3.who.int/icd/vol1htm2003/fr-icd.htm,** be referred to as follows:

Accordingly, malignant neoplasms, stated or presumed to be primary, of specified sites are diseases in the meaning of the present invention:

C00-C14 Lip, oral cavity and pharynx
C15-C26 Digestive organs
C30-C39 Respiratory and intrathoracic organs
C40-C41 Bone and articular cartilage
C43-C44 Skin
C45-C49 Mesothelial and soft tissue
C50 Breast
C51-C58 Female genital organs
C60-C63 Male genital organs
C64-C68 Urinary tract
C69-C72 Eye, brain and other parts of central nervous system
C73-C75 Thyroid and other endocrine glands

C76-C80 Malignant neoplasms of ill-defined, secondary and unspecified sites

C81-C96 Malignant neoplasms, stated or presumed to be primary, of lymphoid, haematopoietic and related tissue

C97 Malignant neoplasms of independent (primary) multiple sites

D00-D09 In situ neoplasms

D10-D36 Benign neoplasms

D37-D48 Neoplasms of uncertain or unknown behaviour [see note before D37]

A preferred group of diseases are the followings
Barret's esophagus, esophageal adenocarcinoma (Stepinac T, Felley C, Jornod P, Lange N, Gabrecht T, Fontolliet C, Grosjean,P, vanMelle G, van den Bergh H, Monnier P, Wagnieres G, Dorta G, (2003) Endoscopic fluorescence detection of intraepithelial neoplasia in Barrett's esophagus after oral administration of aminolevulinic acid. Endoscopy 35: 663-668; Mayinger B, Neidhardt S, Reh H, Martus P, Hahn E (2001) Fluorescence induced with 5-aminolevulinic acid for the endoscopic detection and follow-up of esophageal lesions. Gastrointest Endosc 54: 572-578; Hinnen P, de Rooij F, van Velthuysen M, Edixhoven A, van Hillegersberg R, Tilanus H, Wilson J, Siersema P (1998) Biochemical basis of 5-aminolaevulinic acid-induced protoporphyrin IX accumulation: a study in patients with (pre)malignant lesions of the oesophagus. Br J Cancer 78: 679- 682.)
Basal cell carcinoma (af Klinteberg C, Enejder A, Wang I, Andersson-Engels S, Svanberg S, Svanberg K (1999) Kinetic fluorescence studies of 5-aminolevulinic acid induced protoporphyrin IX accumulation in basal cell carcinomas. J Photochem Photobiol B 49: 120-128;
Bladder cancer (Olivo M, Lau W, Manivasager V, Hoon T, Christopher C (2003) Fluorescence confocal microscopy and image analysis of bladder cancer using 5-aminolevulinic acid. Int J Oncol 22: 523 -528; Kriegmair M, Zaak D, Knuechel R, Baumgartner R, Hofstetter A (1999) 5-Aminolevulinic acid-induced fluorescence endoscopy for the detection of lower urinary tract tumors. Urol Int 63: 27-31, Krieg,R.C., Fickweiler,S., Wolfbeis,O.S. and Knuechel,R. Cell-type specific protoporphyrin IX metabolism in human bladder cancer in vitro. Photochem.Photobiol. 72: 226-233, 2000.
Breast cancer (Peng Q, Moan J, Warloe T, Nesland J, Rimington C (1992) Distribution and photosensitizing efficiency of porphyrins induced by application of exogenous 5-aminolevulinic acid in mice bearing mammary carcinoma. Int J Cancer 52: 433-443; Ponka P (1999) Cell biology of heme. Am J Med Sci 318: 241-256; Sauter E, Ehya H, Klein-Szanto A, Wagner-Mann C, MacGibbon B (2005) Fiberoptic ductoscopy findings in women with and without spontaneous nipple discharge. Cancer 103: 914-921, doi 10.1002/cncr.20865 ; Navone N, Polo C, Frisardi A, Andrade N, Batlle A (1990) Heme biosynthesis in human breast cancer - mimetic 'in vitro' studies ad soe heme enzymic activity levels. Int J Biochem 22: 1407-1411; Ladner D, Steiner R, Allemann J, Haller U, Walt H (2001) Photodynamic diagnosis of breast tumours after oral application of aminolevulinic acid. Br J Cancer 84: 33-37, doi:10.1054/bjoc.2000.1532; Frei K, Bonel H, Frick H, Walt H, Steiner R (2004) Photodynamic detection of diseased axillary sentinel lymph node after oral application of aminolevulinic acid in patients with breast cancer. Br J Cancer 90: 805-809, doi:10.1038/sj.bjc.6601615 ; Dorward,A.M., Fancher,K.S., Duffy,T.M., Beamer,W.G. and Walt,H. Early neoplastic and metastatic mammary tumours of transgenic mice detected by 5-aminolevulinic acid-stimulated protoporphyrin IX accumulation, Br.J.Cancer, 93: 1137-1143, 2005.)
Cervical intrepithetal carcinoma (Bogaards A, Aalders M, Zeyl C, de Blok S, Dannecker C, Hillemanns P, tepp H, Sterenborg H (2002) Localization and staging of cervical intraepithelial neoplasia using double ratio fluorescence imaging. J Biomed Opt 7: 215-220.)
Colorectal carcinoma (Moesta K, Ebert B, Handke T, Nolte D, Nowak C, Haensch W, Pandrey R,Dougherty T, Rinneberg H, Schlag P (2001) Protoporphyrin IX occurs naturally in colorectal cancers and their metastases. Cancer Res 61: 9 91-999 Krieg R, Messmann H, Rauch J, Seeger S, Knuechel R (2002) Metabolic characterization of tumor cell-specific protoporphyrin IX accumulation after exposure to 5-aminolevulinic acid in human colonic cells. Photochem Photobiol 76: 518-525; Endlicher,E., Gelbmann,C.M., Knuchel,R., Furst,A., Szeimies,R.M., Golder,S.K., Scholmerich,J., Lottner, C. and Messmann,H. Hexaminolevulinate-induced fluorescence endoscopy in patients with rectal adenoma and cancer: a pilot study, Gastrointest.Endosc., 60: 449-454, 2004.)
Gastric carcinoma (Moesta T, Kemmner W (unpublished observations).
Glioblastoma (Specific intensity imaging for glioblastoma and neural cell cultures with 5-aminolevulinic acid-derived protoporphyrin IX, J.Neurooncol., 71: 107-111, 2005.)
Leukemia (Chen J, Mak N, Cheung N, Leung R, Peng Q (2001) Endogenous production of protoporphyrin IX induced by 5-aminolevulinic acid in leukemia cells. Acta Pharmacol Sin 22: 163- 168; Furre,I.E., Shahzidi,S., Luksiene,Z., Moller, M.T., Borgen,E., Morgan,J., Tkacz-Stachowska,K., Nesland,J.M. and Peng,Q. Targeting PBR by hexaminolevulinate-mediated photodynamic therapy induces apoptosis through translocation of apoptosis-inducing factor in human leukemia cells, Cancer Res., 65: 11051-11060, 2005; Rebeiz,N., Arkins,S., Kelley,K.W., Durack,G. and Rebeiz,C.A. Modulator of heme biosynthesis induces apoptosis in leukemia cells, J.Clin.Laser Med.Surg., 19: 59-67, 200.)
Lung carcinoma
Melanoma (Bhasin,G., Kausar,H. and Athar,M. Protoporphyrin-IX accumulation and cutaneous tumor regression in mice using a ferrochelatase inhibitor, Cancer Lett., 187: 9-16, 2002.)
Neuroblastoma (Stummer W, Stocker S, Wagner S, Stepp H, Fritsch C, Goetz C, Goetz A, Kiefmann R, Reulen H (1998) Intraoperative detection of malignant gliomas by 5-aminolevulinic acid-induced porphyrin fluorescence. Neurosurgery 42: 518-525; Wu,S.M., Ren,Q.G., Zhou,M.O., Peng,Q. and Chen,J.Y. Protoporphyrin IX production and its photodynamic effects on glioma cells, neuroblastoma cells and normal cerebellar granule cells in vitro with 5-aminolevulinic acid and its hexylester, Cancer Lett., 200: 123-131, 2003.)

Oral cancer (Betz C, Stepp H, Janda P, Arbogast S, Grevers G, Baumgartner R, Leunig A (2002) A comparative study of normal inspection, autofluorescence and 5-ALA-induced PPIX fluorescence for oral cancer diagnosis. Int J Cancer 97: 245-252, doi:10.1002/ijc/.1596.)

Ovarian carcinoma (Loening M, Diddens H, Ku¨pker W, Diedrich K, Hu¨ttmann G (2004) Laparoscopic fluorescence detection of ovarian carcinoma metastases using 5-aminolevulinic acid-induced protoporphyrin IX. Cancer 100: 1650-1656, doi:10.1002/cncr.20155.)

Heptocellular carcinoma (Otake M, Nishiwaki M, Kobayashi Y, Baba S, Kohno E, Kawasaki T, Fujise Y, Nakamura H (2003) Selective accumulation of ALA-induced PpIX and photodynamic effect in chemically induced heptocellular carcinoma. Br J Cancer 89: 730-736, doi:10.1038/sj.bjc.6601135.)

Pancreatic carcinoma

Prostate carcinome

Within the group of inflammatory diseases the following diseases are particularly preferred :

Actinic keratosis (Smits,T., Robles,C.A., van Erp,P.E., van de Kerkhof,P.C. and Gerritsen,M.J. Correlation between macroscopic fluorescence and protoporphyrin IX content in psoriasis and actinic keratosis following application of aminolevulinic acid, J.Invest Dermatol., 125: 833-839, 2005.)

Psoriasis and other skin diseases (Smits,T., Robles,C.A., van Erp,P.E., van de Kerkhof,P.C. and Gerritsen,M.J. Correlation between macroscopic fluorescence and protoporphyrin IX content in psoriasis and actinic keratosis following application of aminolevulinic acid, J.Invest Dermatol., 125: 833-839, 2005. He,D., Behar,S., Nomura,N., Sassa,S., Taketani,S. and Lim,H.W. The effect of porphyrin and radiation on ferrochelatase and 5-aminolevulinic acid synthase in epidermal cells, Photodermatol.Photoimmunol.Photomed., 11: 25-30, 1995.)

Arthritis in its various forms and in particular rheumatoid arthritis (Devesa,I., Ferrandiz,M.L., Guillen,I., Cerda,J.M. and Alcaraz,M.J. Potential role of heme oxygenase-1 in the progression of rat adjuvant arthritis, Lab Invest, 85: 34-44, 2005) and, again in accordance with the above identified international classification:

M05 -M06 rheumatoid arthritis
M07* Psoriatic and enteropathic arthropathies
M08 Juvenile arthritis

A still further group of diseases are proliferative, excematous and papulosquamous diseases of the skin, whereby a preferred disease is psoriasis which is, in accordance with international classification, referred to as

L40 Psoriasis

A still further group of diseases for the treatment and prevention of which the means and methods disclosed herein can be used are diseases which are characterized by hypoxia, more specifically hypoxia in or at the site of the disease, preferably in the tissue which is diseases. Hypoxia is a condition characterized by lack of oxygen which is, among others but not limited thereto, a common ferature of solid carcinomas. Apart from that this is also a rational for the applicability of the technical teaching provided herein for the treatment of the various diseases. Characteristic for hypoxia is a significant increase in ferrochelase expression. This might be responsible for effects such as angiogegensis. Therefore a decrease in ferrochelarase activity might be relevant for the therapeutic approach taught herein. (Liu,Y.L., Ang,S.O., Weigent,D.A., Prchal,J.T. and Bloomer,J.R. Regulation of ferrochelatase gene expression by hypoxia, Life Sci., 75: 2035-2043, 2004.)

Particularly in the light of the above, a still further group of diseases for the treatment and prevention of which the means and methods disclosed herein can be used are diseases which are characterized by angiogenesis, more specifically neo-angiogenesis.

The use of siRNA molecules and RNAi molecules is based on the phenomenon of RNA interferences.

RNA interference refers to the process of sequence specific post-transcriptional gene silencing in animals mediated by short interfering RNAs (siRNAs) (Fire et al., 1998, Nature, 391, 806). The corresponding process in plants is commonly referred to as post-transcriptional gene silecing or RNA silencing and is also referred to as quelling in fungi. The process of post-transcriptional gene silencing is thought to be an evolutionarily-conserved cellular defense mechanism used to prevent the expression of foreign genes which is commonly shared by diverse flora and phyla (Fire et al., 1999, Trends Genet., 15, 358). Such protection from foreign gene expression may have evolved in response to the production of double-stranded RNAs (dsRNAs) derived from viral infection or the random integration of transposon elements into a host genome via a cellular response that specifically destroys homologous single-stranded RNA or viral genomic RNA. The presence of dsRNA in cells triggers the RNAi response though a mechanism that has yet to be fully characterized. This mechanism which is also existing in animal cells and in particular also in mammalian cells, appears to be different from the interferon response that results from dsRNA-mediated activation of protein kinase PKR and 2', 5'-oligoadenylate synthetase resulting in non-specific cleavage of mRNA by ribonuclease L.

The basic design of siRNA molecules or RNAi molecules, which mostly differ in the size, is basically such that the nucleic acid molecule comprises a double-stranded structure. The double-stranded structure comprises a first strand and a second strand. More preferably, the first strand comprises a first stretch of contiguous nucleotides and the second stretch comprises a second stretch of contiguous nucleotides. At least the first stretch and the second stretch are essentially complementary to each other. Such complementarity is typically based on Watson-Crick base pairing or other base-pairing mechanism known to the one skilled in the art, including but not limited to Hoogsteen base-pairing and others. It will be acknowledged by the one skilled in the art that depending on the length of such double-stranded structure a perfect match in terms of base complementarity is not necessarily required. However, such perfect complementarity is preferred in some embodiments. A mismatch is also tolerable, mostly under the proviso that the double-stranded structure is still suitable to trigger the RNA interference mechanism, and that preferably such double-stranded structure is still stably forming under physiological conditions as prevailing in a cell, tissue and organism, respectively, containing or in principle containing such cell, tissue and organ. More preferably, the double-stranded structure is stable at 37 °C in a physiological buffer.

The first stretch, is typically at least partially complementary to a target nucleic acid and the second stretch is, particularly given the relationship between the first and second stretch, respectively, in terms of base complementarity, at least partially identical to the target nucleic acid. The target nucleic acid is preferably an mRNA, although other forms of RNA such as hnRNAs are also suitable for such purpose. Such siRNA molecule and RNAi molecule respectively, is suitable to trigger the RNA interference response resulting in the knock-down of the mRNA for the target molecule. Insofar, this kind of nucleic acid molecule is suitable to decrease the expression of a target molecule by decreasing the expression at the level of mRNA.

Although RNA interference can be observed upon using long nucleic acid molecules comprising several dozens and sometimes even several hundreds of nucleotides and nucleotide pairs, respectively, shorter RNAi molecules are generally preferred. A more preferred range for the length of the first stretch and/or second stretch is from about 18 to 29 consecutive nucleotides, preferably 19 to 25 consecutive nucleotides and more preferably 19 to 23 consecutive nucleotides. More preferably, both the first stretch and the second stretch have the same length. In a further embodiment, the double-stranded structure comprises preferably between 16 and 29, preferably 18 to 25, more preferably 19 to 23 and most preferably 19 to 21 base pairs.

Although in accordance with the present invention, in principle, any part of the mRNA coding for ferrochelatase can be used for the design of such siRNA molecule and RNAi molecule, respectively, the present inventors have surprisingly found that the sequence starting with nucleotide position 381, 1011 and 1980, respectively of the nucleic acid sequence according to SEQ.ID.NO. 1 in downstream, i.e. 3' direction of the nucleic acid sequence according to SEQ.ID.NO. 1 are particularly suitable target regions of the mRNA encoding ferrochelatase. Insofar, any RNAi molecule and siRNA molecule, respectively, targeting these particular regions of ferrochelatase result in a very efficient knock down of ferrochelatase mRNA.

It will be acknowledged by the ones skilled in the art that the particular design of the siRNA molecules and the RNAi molecules, respectively, can vary in accordance with the current and future design principles. For the time being some design principles exist which shall be discussed in more detail in the following and referred to as sub-aspects of the first aspect of the present invention which is related to the nucleic acid molecule according to the present invention. It is within the present invention that all features and embodiments described for one particular sub-aspect, i.e. design of the nucleic acid, is also applicable to any other sub-aspect of the nucleic acid according to the present invention and thus form respective embodiments thereof.

The first sub-aspect is related to nucleic acid according to the present invention, whereby the first stretch comprises a plurality of group of modified nucleotides having a modification at the 2' position, whereby within the stretch each group of modified nucleotides is flanked on one or both sides by a flanking group of nucleotides, whereby the flanking nucleotides forming the flanking group of nucleotides is either an unmodified nucleotide or a nucleotide having a modification different from the modification of the modified nucleotides. Such design is, among others described in international patent application WO 2004/015107. The nucleic acid according to this aspect is preferably a ribonucleic acid although, as will be outlined in some embodiments, the modification at the 2' position results in a nucleotide which as such is, from a pure chemical point of view, no longer a ribonucleotide. However, it is within the present invention that such modified ribonucleotide shall be regarded and addressed herein as a ribonucleotide and the molecule containing such modified ribonucleotide as a ribonucleic acid.

In an embodiment of the ribonucleic acid according to the first sub-aspect of the present invention the ribonucleic acid is blunt ended, either on one side or on both sides of the double-stranded structure. In a more preferred embodiment the double-stranded structure comprises 18 or 19 base pairs. In an even more preferred embodiment, the nucleic acid consists of the first stretch and the second stretch only.

In a further embodiment of the ribonucleic acid according to the first sub-aspect of the present invention said first stretch and/or said second stretch comprise a plurality of groups of modified nucleotides. In a further preferred embodiment the first stretch also comprises a plurality of flanking groups of nucleotides. In a preferred embodiment a plurality of groups means at least two groups.

In another embodiment of the ribonucleic acid according to the first sub-aspect of the present invention said second stretch comprises a plurality of groups of modified nucleotides. In a further preferred embodiment the second stretch also comprises a plurality of flanking groups of nucleotides. In a preferred embodiment a plurality of groups means at least two groups.

In a further preferred embodiment both the first and the second stretch comprise a plurality of both groups of modified nucleotides and flanking groups of nucleotides. In a more preferred embodiment the plurality of both groups of modified nucleotides and flanking groups of nucleotides form a pattern, preferably a regular pattern, on either the first stretch and/or the second stretch, whereby it is even more preferred that such pattern is formed on both the first and the second stretch.

In a preferred embodiment of the ribonucleic acid according to the first sub-aspect of the present invention the group of modified nucleotides and/or the group of flanking nucleotides comprises a number of nucleotides whereby the number is selected from the group comprising one nucleotide to 10 nucleotides. In connection with any ranges specified herein it is to be understood that each range discloses any individual integer between the respective figures used to define the range including said two figures defining said range. In the present case the group thus comprises one nucleotide, two nucleotides, three nucleotides, four nucleotides, five nucleotides, six nucleotides, seven nucleotides, eight nucleotides, nine nucleotides and ten nucleotides.

In another embodiment of the ribonucleic acid according to the first sub-aspect of the present invention the pattern of modified nucleotides of said first stretch is the same as the pattern of modified nucleotides of said second stretch.

In a preferred embodiment of the ribonucleic acid according to the first sub-aspect of the present invention the pattern of said first stretch aligns with the pattern of said second stretch.

In an alternative embodiment of the ribonucleic acid according to the first sub-aspect of the present invention the pattern of said first stretch is shifted by one or more nucleotides relative to the pattern of the second stretch.

In an embodiment of the ribonucleic acid according to the first sub-aspect of the present invention the modification at the 2' position is selected from the group comprising amino, fluoro, methoxy, alkoxy and alkyl.

In another embodiment of the ribonucleic acid according to the first sub-aspect of the present invention the double stranded structure is blunt ended.

In a preferred embodiment of the ribonucleic acid according to the first sub-aspect of the present invention the double stranded structure is blunt ended on both sides of the double-stranded structure.

In another embodiment of the ribonucleic acid according to the first sub-aspect of the present invention the double stranded structure is blunt ended on the double stranded structure's side which is defined by the 5'-end of the first strand and the 3'-end of the second strand.

In still another embodiment of the ribonucleic acid according to the first sub-aspect of the present invention the double stranded structure is blunt ended on the double stranded structure's side which is defined by at the 3'-end of the first strand and the 5'-end of the second strand.

In another embodiment of the ribonucleic acid according to the first sub-aspect of the present invention at least one of the two strands has an overhang of at least one nucleotide at the 5'-end.

In a preferred embodiment of the ribonucleic acid according to the first sub-aspect of the present invention the overhang consists of at least one deoxyribonucleotide.

In a further embodiment of the ribonucleic acid according to the first sub-aspect of the present invention at least one of the strands has an overhang of at least one nucleotide at the 3'-end.

In an embodiment of the ribonucleic acid of the first sub-aspect the length of the double-stranded structure is from about 17 to 21 and more preferably 18 or 19 bases

In another embodiment of the ribonucleic acid of the first sub-aspect the length of said first strand and/or the length of said second strand is independently from each other selected from the group comprising the ranges of from about 15 to about 23 bases, 17 to 21 bases and 18 or 19 bases.

In a preferred embodiment of the ribonucleic acid according to the first sub-aspect the present invention the complementarity between said first strand and the target nucleic acid is perfect.

In an embodiment of the ribonucleic acid according to the first sub-aspect the duplex formed between the first strand and the target nucleic acid comprises at least 15 nucleotides wherein there is one mismatch or two mismatches between said first strand and the target nucleic acid forming said double-stranded structure.

In an embodiment of the ribonucleic acid according to the first sub-aspect both the first strand and the second strand each comprise at least one group of modified nucleotides and at least one flanking group of nucleotides, whereby each group of modified nucleotides comprises at least one nucleotide and whereby each flanking group of nucleotides comprising at least one nucleotide with each group of modified nucleotides of the first strand being aligned with a flanking group of nucleotides on the second strand, whereby the most terminal 5' nucleotide of the first strand is a nucleotide of the group of modified nucleotides, and the most terminal 3' nucleotide of the second strand is a nucleotide of the flanking group of nucleotides.

In a preferred embodiment of the ribonucleic acid according to of the first sub-aspect, each group of modified nucleotides consists of a single nucleotide and/or each flanking group of nucleotides consists of a single nucleotide.

In a further embodiment of the ribonucleic acid according to of the first sub-aspect, on the first strand the nucleotide forming the flanking group of nucleotides is an unmodified nucleotide which is arranged in a 3' direction relative to the nucleotide forming the group of modified nucleotides, and wherein on the second strand the nucleotide forming the group of modified nucleotides is a modified nucleotide which is arranged in 5' direction relative to the nucleotide forming the flanking group of nucleotides.

In a another embodiment of the ribonucleic acid according to the fourth aspect, the first strand comprises eight to twelve, preferably nine to eleven, groups of modified nucleotides, and wherein the second strand comprises seven to eleven, preferably eight to ten, groups of modified nucleotides.

It is within the present invention that what has been specified above is also applicable to the first and second stretch, respectively. This is particular true for those embodiments where the strand consists of the stretch only.

The ribonucleic acid molecule according to such first sub-aspect may be designed is to have a free 5' hydroxyl group, also referred to herein as free 5' OH-group, at the first strand. A free 5' OH-group means that the most terminal nucleotide forming the first strand is present and is thus not modified, particularly not by an end modification. Typically, the terminal 5'-hydroxy group of the second strand, respectively, is also present in an unmodified manner. In a more preferred embodiment, also the 3'-end of the first strand and first stretch, respectively, is unmodified such as to present a free OH-group which is also referred to herein as free 3'OH-group, whereby the design of the 5' terminal nucleotide is the one of any of the afore-described embodiments. Preferably such free OH-group is also present at the 3'-end of the second strand and second stretch, respectively. In other embodiments of the ribonucleic acid molecules as described previously according to the present invention the 3'-end of the first strand and first stretch, respectively, and/or the 3'-end of the second strand and second stretch, respectively, may have an end modification at the 3' end.

As used herein the terms free 5'OH-group and 3'OH-group also indicate that the respective most terminal nucleotide at the 5'end and the 3' end of the polynucleotide, respectively, i.e. either the nucleic acid or the strands and stretches, respectively, forming the double-stranded structure present an OH-group. Such OH-group may stem from either the sugar moiety of the nucleotide, more preferably from the 5'position in case of the 5'OH-group and from the 3'position in case of the 3'OH-group, or from a phosphate group attached to the sugar moiety of the respective terminal nucleotide. The phosphate group may in principle be attached to any OH-group of the sugar moiety of the nucleotide. Preferably, the phosphate group is attached to the 5'OH-group of the sugar moiety in case of the free 5'OH-group and/or to the 3'OH-group of the sugar moiety in case of the free 3'OH-group still providing what is referred to herein as free 5' or 3' OH-group.

As used herein with any embodiment of the first sub-aspect, the term end modification means a chemical entity added to the most 5' or 3' nucleotide of the first and/or second strand. Examples for such end modifications include, but are not limited to, inverted (deoxy) abasics, amino, fluoro, chloro, bromo, CN, CF, methoxy, imidazole, caboxylate, thioate, $C_1$ to $C_{10}$ lower alkyl, substituted lower alkyl, alkaryl or aralkyl, $OCF_3$, OCN, O-, S-, or N-alkyl; O-, S-, or N-alkenyl; $SOCH_3$; $SO_2CH_3$; $ONO_2$; $NO_2$, $N_3$; heterozycloalkyl; heterozycloalkaryl; aminoalkylamino; polyalkylamino or substituted silyl, as, among others, described in European patents EP 0 586 520 B1 or EP 0 618 925 B1.

As used herein, alkyl or any term comprising "alkyl" means any carbon atom chain comprising 1 to 12, preferably 1 to 6 and more, preferably 1 to 2 C atoms.

A further end modification is a biotin group. Such biotin group may preferably be attached to either the most 5' or the most 3' nucleotide of the first and/or second strand or to both ends. In a more preferred embodiment the biotin group is coupled to a polypeptide or a protein. It is also within the scope of the present invention that the polypeptide or protein is attached through any of the other aforementioned end modifications. The polypeptide or protein may confer further characteristics to the inventive nucleic acid molecules. Among others the polypeptide or protein may act as a ligand to another molecule. If said other molecule is a receptor the receptor's function and activity may be activated by the binding ligand. The receptor may show an internalization activity which allows an effective transfection of the ligand bound inventive nucleic acid molecules. An example for the ligand to be coupled to the inventive nucleic acid molecule is VEGF and the corresponding receptor is the VEGF receptor.

Various possible embodiments of the RNAi of the present invention having different kinds of end modification (s) are presented in the following table 1.

**Table 1: Various embodiments of the interfering ribonucleic acid according to the present invention**

| | **1st strand/1st stretch** | **2nd strand/ 2nd** stretch |
|---|---|---|
| **1.) 5'-end** | free OH | free OH |
| **3'-end** | free OH | free OH |

(continued)

| | 1st strand/1st stretch | 2nd strand/ 2nd stretch |
|---|---|---|
| **2.) 5'-end** | free OH | free OH |
| **3'-end** | end modification | end modification |
| **3.) 5'-end** | free OH | free OH |
| **3'-end** | free OH | end modification |
| **4.) 5'-end** | free OH | free OH |
| **3'-end** | end modification | free OH |
| **5.) 5'-end** | free OH | end modification |
| **3'-end** | free OH | free OH |
| **6.) 5'-end** | free OH | end modification |
| **3'-end** | end modification | free OH |
| **7.) 5'-end** | free OH | end modification |
| **3'-end** | free OH | end modification |
| **8.) 5'-end** | free OH | end modification |
| **3'-end** | end modification | end modification |

The various end modifications as disclosed herein are preferably located at the ribose moiety of a nucleotide of the ribonucleic acid. More particularly, the end modification may be attached to or replace any of the OH-groups of the ribose moiety, including but not limited to the 2'OH, 3'OH and 5'OH position, provided that the nucleotide thus modified is a terminal nucleotide. Inverted abasics are nucleotides, either desoxyribonucleotides or ribonucleotides which do not have a nucleobase moiety. This kind of compound is, among others, described in Sternberger et al.(2002), Antisense. Nucl. Ac. Drug Dev. in press.

Any of the aforementioned end modifications may be used in connection with the various embodiments of RNAi depicted in table 1. In connection therewith it is to be noted that any of the RNAi forms or embodiments disclosed herein with the sense strand being inactivated, preferably by having an end modification more preferably at the 5' end, are particularly advantageous. This arises from the inactivation of the sense strand which corresponds to the second strand of the ribonucleic acids described herein, which might otherwise interfere with an unrelated single-stranded RNA in the cell. Thus the expression and more particularly the translation pattern of the transcriptome of a cell is more specifically influenced. This effect is also referred to as off-target effect. Referring to table 1 those embodiments depicted as embodiments 7 and 8 are particularly advantageous in the above sense as the modification results in an inactivation of the - target unspecific - part of the RNAi (which is the second strand) thus reducing any unspecific interaction of the second strand with single-stranded RNA in a cellular or similar system where the RNAi according to the present invention is going to be used to knock down specific ribonucleic acids and proteins, respectively.

In a further embodiment, the nucleic acid according to the first sub-aspect has an overhang at the 5'-end of the ribonucleic acid. More particularly, such overhang may in principle be present at either or both the first strand and second strand of the ribonucleic acid according to the present invention. The length of said overhang may be as little as one nucleotide and as long as 2 to 8 nucleotides, preferably 2, 4, 6 or 8 nucleotides. It is within the present invention that the 5' overhang may be located on the first strand and/or the second strand of the ribonucleic acid according to the present application. The nucleotide(s) forming the overhang may be (a) desoxyribonucleotide(s), (a) ribonucleotide(s) or a continuation thereof.

The overhang preferably comprises at least one desoxyribonucleotide, whereby said one desoxyribonucleotide is preferably the most 5'-terminal one. It is within the present invention that the 3'-end of the respective counter-strand of the inventive ribonucleic acid does not have an overhang, more preferably not a desoxyribonucleotide overhang. Here again, any of the inventive ribonucleic acids may comprise an end modification scheme as outlined in connection with table 1 and/or and end modification as outlined herein.

Taken the stretch of contiguous nucleotides a pattern of modification of the nucleotides forming the stretch may be realised such that a single nucleotide or group of nucleotides which are covalently linked to each other via standard phosphorodiester bonds or, at least partially, through phosphorothioate bonds, show such kind of modification. In case such nucleotide or group of nucleotides which is also referred to herein as group of modified nucleotides, is not forming the 5'-end or 3'-end of said stretch a nucleotide or group of nucleotides follows on both sides of the nucleotide which does not have the modification of the preceding nucleotide or group of nucleotides. It is to be noted that this kind of nucleotide or group of nucleotides, however, may have a different modification. This kind of nucleotide or group of nucleotides is also referred to herein as the flanking group of nucleotides. This sequence of modified nucleotide and group of modified nucleotides, respectively, and unmodified or differently modified nucleotide or group of unmodified or differently modified nucleotides may be repeated one or several times. Preferably, the sequence is repeated more than one time. For reason of clarity the pattern is discussed in more detail in the following, generally referring to a group of modified nucleotides or a group of unmodified nucleotides whereby each of said group may actually comprise as little as a single nucleotide. Unmodified nucleotide as used herein means either not having any of the afore-mentioned modifications at the nucleotide forming the respective nucleotide or group of nucleotides, or having a modification which is different from the one of the modified nucleotide and group of nucleotides, respectively.

It is also within the present invention that the modification of the unmodified nucleotide(s) wherein such unmodified nucleotide(s) is/are actually modified in a way different from the modification of the modified nucleotide(s), can be the same or even different for the various nucleotides forming said unmodified nucleotides or for the various flanking groups of nucleotides.

The pattern of modified and unmodified nucleotides may be such that the 5'-terminal nucleotide of the strand or of the stretch starts with a modified group of nucleotides or starts with an unmodified group of nucleotides. However, in an alternative embodiment it is also possible that the 5'-terminal nuleotide is formed by an unmodified group of nucleotides.

This kind of pattern may be realised either on the first stretch or the second stretch of the interfering RNA or on both. It has to be noted that a 5' phosphate on the target-complementary strand of the siRNA duplex is required for siRNA function, suggesting that cells check the authenticity of siRNAs through a free 5' OH (which can be phosphorylated) and allow only such bona fide siRNAs to direct target RNA destruction (Nykanen, et al. (2001), Cell 107, 309-21).

Preferably, the first stretch shows a kind of pattern of modified and unmodified groups of nucleotides, i. e. of group(s) of modified nucleotides and flanking group(s) of nucleotides, whereas the second stretch does not show this kind of pattern. This may be useful insofar as the first stretch is actually the more important one for the target-specific degradation process underlying the interference phenomenon of RNA so that for specificity reasons the second stretch can be chemically modified so it is not functional in mediating RNA interference.

However, it is also within the present invention that both the first stretch and the second stretch have this kind of pattern. Preferably, the pattern of modification and non-modification is the same for both the first stretch and the second stretch.

In a preferred embodiment the group of nucleotides forming the second stretch and corresponding to the modified group of nucleotides of the first stretch are also modified whereas the unmodified group of nucleotides of or forming the second stretch correspond to the unmodified group of nucleotides of or forming the first stretch. Another alternative is that there is a phase shift of the pattern of modification of the first stretch and first strand, respectively, relative to the pattern of modification of the second stretch and second strand, respectively. Preferably, the shift is such that the modified group of nucleotides of the first strand corresponds to the unmodified group of nucleotides of the second strand and vice versa. It is also within the present invention that the phase shift of the pattern of modification is not complete but overlapping.

In a preferred embodiment the second nucleotide at the terminus of the strand and stretch, respectively, is an unmodified nucleotide or the beginning of group of unmodified nucleotides. Preferably, this unmodified nucleotide or unmodified group of nucleotides is located at the 5'-end of the first and second strand, respectively, and even more preferably of the first strand. In a further preferred embodiment the unmodified nucleotide or unmodified group of nucleotide is located at the 5'-end of the first strand and first stretch, respectively. In a preferred embodiment the pattern consists of alternating single modified and unmodified nucleotides.

In a further preferred embodiment of this aspect of the present invention the interfering ribonucleic acid subject comprises two strands, whereby a 2'-O-methyl modified nucleotide and a non-modified nucleotide, preferably a nucleotide which is not 2'-O-methyl modified, are incorporated on both strands in an alternate manner which means that every second nucleotide is a 2'-O-methyl modified and a non-modified nucleotide, respectively. This means that on the first strand one 2'-O-methyl modified nucleotide is followed by a non-modified nucleotide which in turn is followed by 2'-O-methyl modified nucleotide and so on. The same sequence of 2'-O-methyl modification and non-modification exists on the second strand, whereby there is preferably a phase shift such that the 2'-O-methyl modified nucleotide on the first strand base pairs with a non-modified nucleotide(s) on the second strand and vice versa. This particular arrangement, i. e. base pairing of 2'-O-methyl modified and non-modified nucleotide(s) on both strands is particularly preferred in case of short interfering ribonucleic acids, i. e. short base paired double-stranded ribonucleic acids because it is assumed, although the present inventors do not wish to be bound by that theory, that a certain repulsion exists between two base-pairing 2'-O-methyl modified nucleotides which would destabilise such duplex, preferably short duplexes. About the particular arrangement, it is preferred if the antisense strand starts with a 2'-O-methyl modified nucleotide at the 5' end whereby consequently the second nucleotide is non-modified, the third, fifth, seventh and so on nucleotides are thus again 2'-O-methyl modified whereas the second, fourth, sixth, eighth and the like nucleotides are non-modified nucleotides. Again, not wishing to be bound by any theory, it seems that a particular importance may be ascribed to the second, and optionally fourth, sixth, eighth and/or similar position(s) at the 5' terminal end of the antisense strand which should not comprise any modification, whereas the most 5' terminal nucleotide, i. e. the first 5' terminal nucleotide of the antisense strand may exhibit such modification with any uneven positions such as first, optionally third, fifth and similar position(s) at the antisense strand may be modified. In further embodiments the modification and non-modification, respectively, of the modified and non-modified nucleotide(s), respectively, may be anyone as described herein.

It is within the present invention that the double-stranded structure is formed by two separate strands, i.e. the first and the second strand. However, it is also with in the present invention that the first strand and the second strand are covalently linked to each other. Such linkage may occur between any of the nucleotides forming the first strand and second strand, respectively. However, it is preferred that the linkage between both strands is made closer to one or both ends of the double-stranded structure. Such linkage can be formed by covalent or non-covalent linkages. Covalent linkage may be formed by linking both strands one or several times and at several positions, respectively, by a compound selected from the group comprising methylene blue and bifunctinoal groups. Such bifunctional groups are preferably selected from the group comprising bis(2-chloroethyl)amine, N-acetly-N'-(p-glyoxylbenzoyl)cystamine, 4.thiouracil and psoralene.

In a further embodiment of the ribonucleic acid according to any of the first sub-aspects of the present invention the first strand and the second strand are linked by a loop structure.

In a preferred embodiment of the ribonucleic acid according to the first sub-aspects of the present invention the loop structure is comprised of a non-nucleic acid polymer.

In a preferred embodiment thereof the non-nucleic acid polymer is polyethylene glycol.

In an embodiment of the ribonucleic acid according to any of the first sub-aspects of the present invention the 5'-terminus of the first strand is linked to the 3'-terminus of the second strand.

In a further embodiment of the ribonucleic acid according to any of the aspects of the present invention the 3'-end of the first strand is linked to the 5'-terminus of the second strand.

In an embodiment the loop consists of a nucleic acid. As used herein, LNA as described in Elayadi and Corey (2001) Curr Opin Investig Drugs. 2(4):558-61. Review; Orum and Wengel (2001) Curr Opin Mol Ther. 3(3):239-43; and PNA are regarded as nucleic acids and may also be used as loop forming polymers. Basically, the 5'-terminus of the first strand may be linked to the 3'-terminus of the second strand. As an alternative, the 3'-end of the first strand may be linked to the 5'-terminus of the second strand. The nucleotide sequence forming said loop structure is regarded as in general not being critical. However, the length of the nucleotide sequence forming such loop seems to be critical for sterical reasons. Accordingly, a minimum length of four nucleotides seems to be appropriate to form the required loop structure. In principle, the maximum number of nucleotides forming the hinge or the link between both stretches to be hybridized is not limited. However, the longer a polynucleotide is, the more likely secondary and tertiary structures are formed and thus the required orientation of the stretches affected. Preferably, a maximum number of nucleotides forming the hinge is about 12 nucleotides. It is within the disclosure of this application that any of the designs described above may be combined with the present sixth strategy, i. e. by linking the two strands covalently in a manner that back folding (loop) can occur through a loop structure or similar structure.

The present inventors have surprisingly found that if the loop is placed 3' of the antisense strand, i. e. the first strand of the ribonucleic acid(s) according to the present invention, the activities of this kind of RNAi are higher compared to the placement of the loop 5' of the antisense strand. Accordingly, the particular arrangement of the loop relative to the antisense strand and sense strand, i. e. the first strand and the second strand, respectively, is crucial and is thus in contrast to the understanding as expressed in the prior art where the orientation is said to be of no relevance. However, this seems not true given the experimental results presented herein. The understanding as expressed in the prior art is based on the assumption that any RNAi, is subject to a processing during which non-loop linked RNAi is generated. However, if this was the case, the clearly observed increased activity of those structures having the loop placed 3' of the antisense could not be explained. Insofar a preferred arrangement in 5' $\rightarrow$ 3' direction of this kind of small interfering RNAi is second strand - loop - first strand. The respective constructs may be incorporated into suitable vector systems. Preferably the vector comprises a promoter for the expression of RNAi. Preferably the respective promoter is pol III and more preferably the promoters are the U6, H1, 7SK promoter as described in Good et al. (1997) Gene Ther, 4, 45-54.

The second sub-aspect of the first aspect of the present invention is related to a nucleic acid according to the present invention, whereby first stretch and/or the second stretch comprise at the 3' end a dinucleotide, whereby such dinucleotide is preferably TT. In a preferred embodiment, the length of the first stretch and/or of the second stretch consists of either 19 or 21 nucleotides and more preferably the double-stranded structure comprises 18 to 22 and more preferably 19 to 21 base pairs. The design of the nucleic acid in accordance with this sub-aspect is described in more detail in e.g., in international patent application WO O1/75164.

The third sub-aspect of the first aspect of the present invention is related to a nucleic acid according to the present invention, whereby the first and/or the second stretch comprise an overhang of 1 to 5 nucleotides at the 3' end. The design of the nucleic acid in accordance with this sub-aspect is described in more detail in international patent application WO02/44321. More preferably such overhang is a ribonucleic acid. In a preferred embodiment the each of the strands and more preferably each of the stretches as defined herein has a length from 19 to 25 nucleotides, whereby more preferably the strand consists of the stretch. In a preferred embodiment, the double-stranded structure of the nucleic acid according to the present invention comprises 17 to 25 base pairs, preferably 19 to 21 base pairs.

The fourth sub-aspect of the first aspect of the present invention is related to a nucleic acid according to the present invention, whereby the first and/or the second stretch comprise an overhang of 1 to 5 nucleotides at the 3' end. The design of the nucleic acid in accordance with this sub-aspect is described in

In a fifth sub-aspect of the first aspect of the present invention the nucleic acid according to the present invention is a nucleic acid which is a chemically synthesized double-stranded short interfering nucleic acid (siNA) molecule which directs cleavage of a ferrochalatase RNA, preferably via RNA interference, wherein each strand of said siNA molecule is 18 to 27 or 19 to 29 nucleotides in length and said siNa molecule comprises at least one chemically modified nucleotide non-nucleotide. The design of the nucleic acid in accordance with this sub-aspect is described in more detail in international patent application WO03/070910 and UK patent 2 397 062.

In one embodiment thereof the siNA molecule comprises no ribonucleotides. In another embodiment, the siNA molecule comprises one or more nucleotides. In another embodiment chemically modified nucleotide comprises a 2'-deoxy nucleotide. In another embodiment chemically modified nucleotide comprises a 2'-deoxy-2'-fluoro nucleotide. In another embodiment chemically modified nucleotide comprises a 2'-O-methyl nucleotide. In another embodiment chemically modified nucleotide comprises a phosphorothioate internucleotide linkage. In a further embodiment the non-nucleotide comprises an abasic moiety, whereby preferably the abasic moiety comprises an inverted deoxyabasic moiety. In another embodiment non-nucleotide comprises a glyceryl moiety.

In a further embodiment, the first strand and the second strand are connected via a linker molecule. Preferably, the linker molecule is polynucleotide linker. Alternatively, the linker molecule is a non-nucleotide linker.

In a further embodiment of the nucleic acid according to the fifth sub-aspect, the pyrimidine nucleotides in the second strand are 2'-O-methyl pyrimidine nucleotides.

In a further embodiment of the nucleic acid according to the fifth sub-aspect, the purine nucleotides in the second strand are 2'-deoxy purine nucleotides.

In a further embodiment of the nucleic acid according to the fifth sub-aspect, the pyrimidine nucleotides in the second strand are 2'-deoxy-2'-fluoro pyrimidine nucleotides.

In a further embodiment of the nucleic acid according to the fifth sub-aspect, the second strand includes a terminal cap moiety at the 5' end, the 3' end or both the 5' end and the 3' end.

In a further embodiment of the nucleic acid according to the fifth sub-aspect, the pyrimidine nucleotides in the first strand are 2'-deoxy-2' fluoro pyrimidine nucleotides.

In a further embodiment of the nucleic acid according to the fifth sub-aspect, the purine nucleotides in the first strand are 2'-O-methyl purine nucleotides.

In a further embodiment of the nucleic acid according to the fifth sub-aspect, the purine nucleotides in the first strand are 2'-deoxy purine nucleotides.

In a further embodiment of the nucleic acid according to the fifth sub-aspect, the first strand comprises a phosphorothioate internucleotide linkage at the 3' end of the first strand. In a further embodiment of the nucleic acid according to the fifth sub-aspect, the first strand comprises a glyceryl modification ar the 3' end of the first strand.

In a further embodiment of the nucleic acid according to the fifth sub-aspect, about 19 nucleotides of both the first and the second strand are base-paired and wherein preferably at least two 3' terminal nucleotides of each strand of the siNA molecule are not base-paired to the nucleotides of the other strand. Preferably, each of the two 3' terminal nucleotides of each strand of the siNA molecule are 2'-deoxy-pyrimidines. More preferably, the 2'deoxy-pyrimidine is 2' deoxy-thymidine.

In a further aspect of the nucleic acid according to the fifth sub-aspect, the 5' end of the first strand comprises a phosphate group.

In one embodiment particularly of the fifth sub-aspect of the nucleic acid according to the present invention, a siNA molecule of the invention comprises modified nucleotides while maintaining the ability to mediate RNAi. The modified nucleotides can be used to improve in *vitro* or *in vivo* characteristics such as stability, activity, and/or bioavailability. For example, a siNA molecule of the invention can comprise modified nucleotides as a percentage of the total number of nucleotides present in the siNA molecule. As such, a siNA molecule of the invention can generally comprise about 5 % to about 100 % modified nucleotides (e. g., 5 %, 10 %, 15 %, 20 %,25 %, 30 %,35 %,40 %, 45 %, 50 %, 55 %, 60 %, 65 %, 70 %, 75 %, 80 %, 85 %, 90 %, 95 % or 100 % modified nucleotides). The actual percentage of modified nucleotides present in a given siNA molecule will depend on the total number of nucleotides present in the siNA. If the siNA molecule is single stranded, the percent modification can be based upon the total number of nucleotides present in the single stranded siNA molecules. Likewise, if the siNA molecule is double stranded, the percent modification can be based upon the total number of nucleotides present in the sense strand, antisense strand, or both the sense and antisense strands.

In a non-limiting example, the introduction of chemically-modified nucleotides into nucleic acid molecules particularly of the fifth sub-aspect of the nucleic acid according to the present invention provides a powerful tool in overcoming potential limitations of *in vivo* stability and bioavailability inherent to native RNA molecules that are delivered exogenously. For example, the use of chemically-modified nucleic acid molecules can enable a lower dose of a particular nucleic acid molecule for a given therapeutic effect since chemically-modified nucleic acid molecules tend to have a longer half-life in serum. Furthermore, certain chemical modifications can improve the bioavailability of nucleic acid molecules by targeting particular cells or tissues and/or improving cellular uptake of the nucleic acid molecule. Therefore, even if the activity of a chemically-modified nucleic acid molecule is reduced as compared to a native nucleic acid molecule, for example, when compared to an all-RNA nucleic acid molecule, the overall activity of the modified nucleic acid molecule can be greater than that of the native molecule due to improved stability and/or delivery of the molecule. Unlike native unmodified siNA, chemically-modified siNA can also minimize the possibility of activating interferon activity in humans.

Preferably in connection with the fifth sub-aspect of the nucleic acid according to the present invention, the antisense strand, i.e. the first strand, of a siNA molecule of the invention can comprise a phosphorothioate internucleotide linkage at the 3'-end of said antisens region. The antisense strand can comprise about one to about five phosphorothioate internucleotide linkages at the 5'-end of said antisense region. The 3'-terminal nucleotide overhangs of a siNA molecule of the invention can comprise ribonucleotides or deoxyribonucleotides that are chemically-modified at a nucleic acid sugar, base or backbone. The 3'-terminal nucleotide overhangs can comprise one or more universal base ribonucleotides. The 3'-terminal nucleotide overhangs can comprise one or more acyclic nucleotides.

It will be acknowledged by the ones skilled in the art that particularly the embodiment of the present invention which comprises a loop made of nucleotides is suitable to be used and expressed by a vector. Preferably, the vector is an expression vector. Such expression vector is particular useful in any gene therapeutic approach. Accordingly, such vector can be used for the manufacture of a medicament which is preferable to be used for the treatment of the diseases disclosed herein. It will, however, also be acknowledged by the ones skilled in the art that any embodiment of the nucleic acid according to the present invention which comprises any non-naturally occurring modification cannot immediately be used for expression in a vector and an expression system for such vector such as a cell, tissue, organ and organism. However, it is within the present invention that the modification may be added to or introduced into the vector derived or vector expressed nucleic acid according to the present invention, after the expression of the nucleic assay by the vector. A particularly preferred vector is a plasmid vector or a viral vector. The technical teaching on how to use siRNA molecules and RNAi molecules in an expression vector is, e.g., described in international patent application WO O1/70949. It will be acknowledged by the ones skilled in the art that such vector is preferably useful in any method either therapeutic or diagnostic where a sustained presence of the nucleic acid according to the present invention is desired and useful, respectively, whereas the non-vector nucleic acid according to the present invention and in particular the chemically modified or chemically synthesized nucleic acid according to the present invention is particularly useful where the transient presence of the molecule is desired or useful.

In a further aspect, the present invention is related to a cell, preferably an isolated cell which is in a more preferred embodiment a human isolated cell, whereby such cell contains a nucleic acid and vector, respectively, according to the present invention, either transiently or permanently. In a further aspect, the invention is related to a tissue, organ or animal, whereby the animal is preferably a mammal, more preferably excluding a human being, whereby such animal contains a nucleic acid, vector, and cell, respectively according to the present invention.

The synthesis of nucleic acid greater than 100 nucleotides in length is difficult using automated methods, and the therapeutic cost of such molecules is prohibitive. In this invention, small nucleic acids or nucleic acid motifs ("small" preferably refers to nucleic acid motifs no more than 100 nucleotides in length, preferably no more than 80 nucleotides in length, and most preferably no more than 50 nucleotides in length; e. g., individual siNA or siRNA oligonucleotide sequences or siNA or siRNA sequences synthesized in tandem) are preferably used for exogenous delivery. The simple structure of these molecules increases the ability of the nucleic acid to invade targeted regions of protein and/or RNA structure. Exemplary molecules of the instant invention are chemically synthesized, and others can similarly be synthesized.

Oligonucleotides (e. g., certain modified oligonucleotides or portions of oligonucleotides lacking ribonucleotides) are synthesized using protocols known in the art, for example as described in Caruthers et al., 1992, Methods in Enzymology 211, 3-19, Thompson et al., International PCT Publication No. WO 99/54459, Wincott et al., 1995, Nucleic Acids Res. 23, 2677-2684, Wincott et al., 1997, Methods Mol. Bio., 74, 59, Brennan et al., 1998, Biotechnol Bioeng., 61, 33-45, and Brennan, U.S. Pat. No. 6,001,311. All of these references are incorporated herein by reference. The synthesis of oligonucleotides makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 μmol scale protocol with a 2.5 min coupling step for 2'-O-methylated nucleotides and a 45 sec coupling step for 2'-deoxy nucleotides or 2'-deoxy-2'-fluoro nucleotides. The amounts and the contact times of the reagents used in the synthesis cycle are detailed, e.g., in UK patent 2 397 062. Alternatively, syntheses at the 0.2 μmol scale can be performed on a 96-well plate synthesizer, such as the instrument produced by Protogene (Palo Alto, CA) with minimal modification to the cycle. A 33-fold excess (60 μL of 0.11 M = 6.6 μmol) of 2'-O-methyl phosphoramidite and a 105-fold excess of S-ethyl tetrazole (60 μL of 0.25 M = 15 μmol) can be used in cach coupling cycle of 2'-O-methyl residues relative to polymer-bound 5'-hydroxyl. A 22-fold excess (40 μL of 0.11 M = 4.4 μmol) of deoxy phosphoramidite and a 70-fold excess of S-ethyl tetrazole (40 μL of 0.25 M = 10 μmol) can be used in each coupling cycle of deoxy residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% *N*-methyl imidazole in THF (ABI) and 10% acetic anhydride/10% 2,6-lutidine in THF (ABI); and oxidation solution is 16.9 mM $I_2$, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide, 0.05 M in acetonitrile) is used.

Deprotection of the DNA-based oligonucleotides is performed as follows: the polymer-bound trityl-on oligoribonucleotide is transferred to a 4 mL glass screw top vial and suspended in a solution of 40% aq. methylamine (1mL) at 65°C for 10 min. After cooling to 20°C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder.

The method of synthesis used for RNA including certain siRNA molecules of the invention follows the procedure as described in Usman et al., 1987, J Am. Chem. Soc., 109, 7845; Scaringe et al., 1990, Nucleic Acids Res., 18, 5433; and Wincott et al., 1995, Nucleic Acids Res. 23, 2677-2684 Wincott et al., 1997, Methods Mol. Bio., 74, 59, and makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 μmol scale protocol with a 7.5 min coupling step for alkylsilyl protected nucleotides and a 2.5 min coupling step for 2'-O-methylated nucleotides. The amounts and the contact times of the reagents used in the synthesis cycle are detailed, e.g., in UK patent 2 397 062. Alternatively, syntheses at the 0.2 μmol scale can be done on a 96-well plate synthesizer, such as the instrument produced by Protogene (Palo Alto, CA) with minimal modification to the cycle. A 33-fold excess (60 μL of 0.11 M = 6.6 μmol) of 2'-O-methyl phosphoramidite and a 75-fold excess of S-ethyl tetrazole (60 μL of 0.25 M = 15 μmol) can be used in each coupling cycle of 2'-O-methyl residues relative to polymer-bound 5'-hydroxyl. A 66-fold excess (120 μL of 0.11 M = 13.2 μmol) of alkylsilyl (ribo) protected phosphoramidite and a 150-fold excess of S-ethyl tetrazole (120 μL of 0.25 M = 30 μmol) can be used in each coupling cycle of ribo residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% *N*-methyl imidazole in THF (ABI) and 10% acetic anhydride/10% 2,6-lutidine in THF (ABI); oxidation solution is 16.9 mM $I_2$, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beacage reagent (311-1,2-Benzodithiol-3-one 1,1-dioxide 0.05 M in acetonitrile) is used.

Deprotection of the RNA is performed using either a two-pot or one-pot protocol. For the two-pot protocol, the polymer-bound trityl-on oligoribonucleotide is transferred to a 4 mL glass screw top vial and suspended in a solution of 40% aq. methylamine (1 mL) at 65°C for 10 min. After cooling to -20°C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. The base deprotected oligoribonucleotide is resuspended in anhydrous TEA/HF/NMP solution (300 μΛ of a solution of 1.5 μL N-methylpyrrolidinone, 750 μL TEA and 1 mL TEA 3HF to provide a 1.4 M HF concentration) and heated to 65°C. After 1.5 h, the oligomer is quenched with 1.5 M $NH_4HCO_3$.

Alternatively, for the one-pot protocol, the polymer-bound trityl-on oligoribonucleotide is transferred to a 4 ml glass screw top vial and suspended in a solution of 33% ethanolic methylamine/DMSO: 1/1 (0.8 mL) at 65°C for 15 min. The vial is brought to room temperature TEA 3HF (0.1 mL) is added and the vial is heated at 65°C for 15 min. The sample is cooled at -20°C and then quenched with 1.5 M $NH_4HCO_3$.

For purification of the trityl-on oligomers, the quenched $NH_4HCO_3$ solution is loaded onto a C-18 containing cartridge that had been prewashed with acetonitrile followed by 50 mM TEAA. After washing the loaded cartridge with water, the RNA is detritylated with 0.5% TFA for 13 min. The cartridge is then washed again with water, salt exchanged with 1 M NaCl and washed with water again. The oligonucleotide is then eluted with 30% acctonitrile.

The average stepwise coupling yields are typically >98% (Wincott et al., 1995 Nucleic Acids Res. 23, 2677-2684). Those of ordinary skill in the art will recognize that the scale of synthesis can be adapted to be larger or smaller than the example described above including but not limited to 96-well format.

Alternatively, the nucleic acid molecules of the present invention can be synthesized separately and joined together post-synthetically, for example, by ligation (Moore et al., 1992, Science 256, 9923; Draper et al., International PCT publication No. WO 93/23569; Shabarova et al., 1991, Nucleic Acids Research 19, 4247; Bellon et al., 1997, Nucleosides & Nucleotides, 16, 951; Bellon et al., 1997, Bioconjugate Chem. 8, 204), or by hybridization following synthesis and/or deprotection.

The nucleic acid molecules of the invention can also be synthesized via a tandem synthesis methodology as described in Example 1 of UK patent 2 397 062, wherein both strands are synthesized as a single contiguous oligonucleotide fragment or strand separated by a cleavage linker which is subsequently cleaved to provide separate nucleic acid fragments or strands that hybridize and permit purification of the double-stranded structure. The linker can be a polynucleotide linker or a non-nucleotide linker. The tandem synthesis of the nucleic acid as described herein can be readily adapted to both multiwell/multiplate synthesis platforms such as well or similarly larger multi-well platforms. The tandem synthesis of siNA as described herein can also be readily adapted to large scale synthesis platforms employing batch reactors, synthesis columns and the like.

A nucleic acid molecule according to the present invention can also be assembled from two distinct nucleic acid strands or fragments wherein one fragment includes the first strand and the second fragment includes the second region of the nucleic acid molecule.

The nucleic acid molecules of the present invention can be modified extensively to enhance stability by modification with nuclease resistant groups, for example, 2'-amino, 2'-C-allyl, 2'-fluoro, 2'-O-methyl, 2'-H (for a review see Usman and Cedergren, 1992, TIBS 17, 34; Usman et al., 1994, Nucleic Acids Symp. Ser. 31, 163). The nucleic acid molecules can be purified by gel electrophoresis using general methods or cn be purified by high pressure liquid chromatography (HPLC; see Wincott et al., supra, the totality of which is hereby incorporated herein by reference) and re-suspended in water.

In another aspect of the invention and as already briefly addressed above, nucleic acid molecules of the invention can be expressed from transcription units inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. The recombinant vectors capable of expressing the nucleic molecules can be delivered as described herein, and persist in target cells. Alternatively, viral vectors can be used that provide for transient expression of siNA molecules.

As already described above, chemically synthesizing nucleic acid molecules with modifications (base, sugar and/or phosphate) can prevent their degradation by serum ribonucleases, which can increase their potency (see, e.g., Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; Usman et al., International Publication No. WO 91/03162; Sproat, U.S. Pat. No. 5,334,711; Gold et al., U.S. Pat. No. 6,300,074; and Burgin et al., supra; all of which are incorporated by reference herein). All of the above references describe various chemical modifications that can be made to the base, phosphate and/or sugar moieties of the nucleic acid molecules described herein. Modifications that enhance their efficacy in cells, and removal of bases from nucleic acid molecules to shorten oligonucleotide synthesis times and reduce chemical requirements are desired.

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-C-allyl, 2'-fluoro, 2'-O-methyl, 2'-O-allyl, 2'-H, nucleotide base modifications (for a review see Usman and Cedergren, 1992, TIBS. 17, 34; Usman et al., 1994, Nucleic Acids Symp. Ser. 31, 163; Burgin et al., 1996, Biochemistry, 35, 14090). Sugar modification of nucleic acid molecules have been extensively described in the art (see Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci., 1992, 17, 334-339; Usman et al. International Publication PCT No. WO 93/15187; Sproat, U.S. Pat. No. 5,334,711 and Beigelman et al., 1995, J. Biol. Chem., 270, 25702; Beigelman et al., International PCT publication No. WO 97/26270; Beigelman et al., U.S. Pat. No. 5,716,824; Usman et al., U.S. Pat. No. 5,627,053; Woolf et al., International PCT Publication No. WO 98/13526; Thompson et al., USSN 60/082,404 which was filed on April 20, 1998; Karpeisky et al., 1998, Tetrahedron Lett., 39, 1131; Eamshaw and Gait, 1998, Biopolymers (Nucleic Acid Sciences), 48, 39-55; Verma and Eckstein, 1998, Annu. Rev. Biochem., 67, 99-134; and Burlina et al., 1997, Bioorg. Med. Chem., 5, 1999-2010; all of the references are hereby incorporated in their totality by reference herein). Such publications describe general methods and strategies to determine the location of incorporation of sugar, base and/or phosphate modifications and the like into nucleic acid molecules without modulating catalysis, and are incorporated by reference herein. In view of such teachings, similar modifications can be used as described herein to modify the nucleic acid molecules of the instant invention so long as the ability of such nucleic acid molecules to promote RNAi is cells is not significantly inhibited.

While chemical modification of oligonucleotide internucleotide linkages with phosphorothioate, phosphorodithioate, and/or 5'-methylphosphonate linkages improves stability, excessive modifications can cause some toxicity or decreased activity. Therefore, when designing nucleic acid molecules, the amount of these internucleotide linkages should be minimized. The reduction in the concentration of these linkages should lower toxicity, resulting in increased efficacy and higher specificity of these molecules.

Short interfering nucleic acid molecules which are an embodiment of the nucleic acid molecules according to the present invention and which are also referred to as siNA molecules, having chemical modification that maintain or enhance activity are provided. Such a nucleic acid is also generally more resistant to nucleases than an unmodified nucleic acid. Accordingly, the in vitro and/or in vivo activity should not be significantly lowered. In cases in which modulation is the goal, therapeutic nucleic acid molecules delivered exogenously should optimally be stable within cells until translation of the target RNA has been modulated long enough to reduce the levels of the undesirable protein. This period of time varies between hours to days depending upon the disease state. Improvements in the chemical synthesis of RNA and DNA (Wincott et al., 1995, Nucleic Acids Res. 23, 2677; Caruthers et al., 19922, Methods in Enzymology 211,3-19 (incorporated by reference herein)) have expanded the ability to modify nucleic acid molecules by introducing nucleotide modifications to enhance their nuclease stability, as described above.

In one embodiment, nucleic acid molecules of the invention include one or more (e.g., about 1, 2, 3, 4, 5, 6, 7, 8, 9, 10, or more) G-clamp nucleotides. A G-clamp nucleotide is a modified cytosine analog wherein the modifications confer the ability to hydrogen bond both Watson-Crick and Hoogsteen faces of a complementary guanine within a duplex, see for example Lin and Matteucci, 1998, J. Am. Chem. Soc., 120, 8531-8532. A single G-clamp analog substitution within an oligonucleotide can result in substantially enhanced helical thermal stability and mismatch discrimination when hybridized to complementary oligonucleotides. The inclusion of such nucleotides in nucleic acid molecules of the invention results in both enhanced affinity and specificity to nucleic acid targets, complementary sequences, or template strands. In another embodiment, nucleic acid molecules of the invention include one or more (e.g., about 1, 2, 3, 4, 5, 6, 7, 8, 9, 10, or more) LNA "locked nucleic acid" nucleotides such as a 2', 4'-C methylene bicycle nucleotide (see for example Wengel et al., International PCT Publication No. WO 00/66604 and WO 99/14226).

In another embodiment, the invention features conjugates and/or complexes of siNA molecules of the invention. Such conjugates and/or complexes can be used to facilitate delivery of nucleic acid molecules into a biological system, such as a cell. The conjugates and complexes provided by the instant invention can impart therapeutic activity by transferring therapeutic compounds across cellular membranes, altering the pharmacokinetics, and/or modulating the localization of nucleic acid molecules of the invention. The present invention encompasses the design and synthesis of novel conjugates and complexes for the delivery of molecules, including, but not limited to, small molecules, lipids, phospholipids, nucleosides, nucleotides, nucleic acids, antibodies, toxins, negatively charged polymers and other polymers, for example proteins, peptides, hormones, carbohydrates, polyethylene glycols, or polyamines, across cellular membranes. In general, the transporters described are designed to be used either individually or as part of a multi-component system, with or without degradable linkers. These compounds are expected to improve delivery and/or localization of nucleic acid molecules of the invention into a number of cell types originating from different tissues, in the presence or absence of serum (see Sullenger and Cech, u.S. Pat. No. 5,854,038). Conjugates of the molecules described herein can be attached to biologically active molecules via linkers that are biodegradable, such as biodegradable nucleic acid linker molecules.

The term "biodegradable linker" as used herein, refers to a nucleic acid or non-nucleic acid linker molecule that is designed as a biodegradable linker to connect one molecule to another molecule, for example, a biologically active molecule to a nucleic acid molecule of the invention. The biodegradable linker is designed such that its stability can be modulated for a particular purpose, such as delivery to a particular tissue or cell type. The stability of a nucleic acid-based biodegradable linker molecule can be modulated by using various chemistries, for example combinations of ribonucleotides, deoxyribonucleotides, and chemically-modified nucleotides, such as 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example, an oligonucleotide of about 2, 3, 4, 5, 6, 7, 8, 9, 10, 11, 12, 13, 14, 15, 16, 17, 18, 19, or 20 nucleotides in length, or can comprise a single nucleotide with a phosphorus-based linkage, for example, a phosphoramidate or phosphodiester linkage. The biodegradable nucleic acid linker molecule can also comprise nucleic acid backbone, nucleic acid sugar, or nucleic acid base modifications.

The term "biodegradable" as used herein, refers to degradation in a biological system, for example enzymatic degradation or chemical degradation.

The term "biologically active molecule" as used herein, refers to compounds or molecules that are capable of eliciting or modifying a biological response in a system. Non-limiting examples of biologically active nucleic acid molecules either alone or in combination with other molecules contemplated by the instant invention include therapeutically active molecules such as antibodies, hormones, antivirals, peptides, proteins, chemotherapeutics, small molecules, vitamins, co-factors, nucleosides, nucleotides, oligonucleotides, enzymatic nucleic acids, antisense nucleic acids, triplex forming oligonucleotides, 2,5-A chimeras, siNA, dsRNA, allozymes, aptamers, decoys and analogs thereof. Biologically active molecules of the invention also include molecules capable of modulating the pharmacokinetics and/or pharmacodynamics of other biologically active molecules, for example, lipids and polymers such as polyamines, polyamides, polyethylene glycol and other polyethers.

The term "phospholipids" as used herein, refers to a hydrophobic molecule comprising at least one phosphorus group. For example, a phospholipid can comprise a phosphorus-containing group and saturated or unsaturated alkyl group, optionally substituted with OH, COOH, oxo, amine, or substituted or unsubstituted aryl groups.

Nucleic acid molecules according to the present invention which are delivered exogenously optimally are stable within cells until reverse transcription of the RNA has been modulated long enough to reduce the levels of the RNA transcript. The nucleic acid molecules are resistant to nucleases in order to function as effective intracellular therapeutic agents or diagnostic agents or diagnostic means. Improvements in the chemical synthesis of nucleic acid molecules described in the instant invention and in the art have expanded the ability to modify nucleic acid molecules by introducing nucleotide modifications to enhance their nuclease stability as described above.

In yet another embodiment, siNA molecules having chemical modifications that maintain or enhance enzymatic activity of proteins involved in RNAi are provided. Such nucleic acids are also generally more resistant to nucleases than unmodified nucleic acids. Thus, *in vitro* and/or *in vivo* the activity should no the significantly lowered.

In a still further aspect the present invention is related to the use of ribozymes and antisense molecules in general which are directed to the same regions of the mRNA and corresponding positions on the genomic DNA of the target molecule, i.e. PpIX. From a practical point of view such antisense molecules and ribozymes, respectively thus also comprise a strand of the siRNA molecules of the present invention, more preferably the strand which is referred to as the antisense strand. The generation of antisense molecules and ribozyme is known to the ones skilled in the art.

Basically, antisense oligonucleotides hybridise based on base complementarity, with a target RNA, preferably with a mRNA, thereby activate RNase H. RNase H is activated by both phosphodiester and phosphorothioate-coupled DNA. Phosphodiester-coupled DNA, however, is rapidly degraded by cellular nucleases with the exception of phosphorothioate-coupled DNA. These resistant, non-naturally occurring DNA derivatives do not inhibit RNase H upon hybridisation with RNA. In other words, antisense polynucleotides are only effective as DNA RNA hybride complexes. Examples for this kind of antisense oligonucleotides are described, among others, in US-patent US 5,849,902 and US 5,989,912. In other words, based on the nucleic acid sequence of the target molecule which in the present case is the nucleic acid coding for protein kinase N beta, either from the target protein from which a respective nucleic acid sequence may in principle be deduced, or by knowing the nucleic acid sequence as such, particularly the mRNA, suitable antisense oligonucleotides may be designed base on the principle of base complementarity.

Particularly preferred are antisense-oligonucleotides which have a short stretch of phosphorothioate DNA (3 to 9 bases). A minimum of 3 DNA bases is required for activation of bacterial RNase H and a minimum of 5 bases is required for mammalian RNase H activation. In these chimeric oligonucleotides there is a central region that forms a substrate for RNase H that is flanked by hybridising "arms" comprised of modified nucleotides that do not form substrates for RNase H. The hybridising arms of the chimeric oligonucleotides may be modified such as by 2'-O-methyl or 2'-fluoro. Alternative approaches used methylphosphonate or phosphoramidate linkages in said arms. Further embodiments of the antisense oligonucleotide useful in the practice of the present invention are P-methoxyoligonucleotides, partial P-methoxyoligodeoxyribonucleotides or P-methoxyoligonucleotides.

Of particular relevance and usefulness for the present invention are those antisense oligonucleotides as more particularly described in the above two mentioned US patents. These oligonucleotides contain no naturally occurring 5'→3'-linked nucleotides. Rather the oligonucleotides have two types of nucleotides: 2'-deoxyphosphorothioate, which activate RNase H, and 2'-modified nucleotides, which do not. The linkages between the 2'-modified nucleotides can be phosphodiesters, phosphorothioate or P-ethoxyphosphodiester. Activation of RNase H is accomplished by a contiguous RNase H-activating region, which contains between 3 and 5 2'-deoxyphosphorothioate nucleotides to activate bacterial RNase H and between 5 and 10 2'- deoxyphosphorothioate nucleotides to activate eucaryotic and, particularly, mammalian RNase H. Protection from degradation is accomplished by making the 5' and 3' terminal bases highly nuclease resistant and, optionally, by placing a 3' terminal blocking group.

More particularly, the antisense oligonucleotide comprises a 5' terminus and a 3' terminus; and from 11 to 59 5'→3'-linked nucleotides independently selected from the group consisting of 2'-modified phosphodiester nucleotides and 2'-modified P-alkyloxyphosphotriester nucleotides; and wherein the 5'-terminal nucleoside is attached to an RNase H-activating region of between three and ten contiguous phosphorothioate-linked deoxyribonucleotides, and wherein the 3'-terminus of said oligonucleotide is selected from the group consisting of an inverted deoxyribonucleotide, a contiguous stretch of one to three phosphorothioate 2'-modified ribonucleotides, a biotin group and a P-alkyloxyphosphotriester nucleotide.

Also an antisense oligonucleotide may be used wherein not the 5' terminal nucleoside is attached to an RNase H-activating region but the 3' terminal nucleoside as specified above. Also, the 5' terminus is selected from the particular group rather than the 3' terminus of said oligonucleotide.

Suitable and useful antisense oligonucleotides are also those comprising a 5' terminal RNase H activating region and having between 5 and 10 contiguous deoxyphosphorothioate nucleotides; between 11 to 59 contiguous 5'→3'-linked 2'-methoxyribonucleotides; and an exonuclease blocking group present at the 3' end of the oligonucleotide that is drawn from the group consisting of a non-5'-3'-phosphodiester-linked nucleotide, from one to three contiguous 5'-3'-linked modified nucleotides and a non-nucleotide chemical blocking group.

Two classes of particularly preferred antisense oligonucleotides can be characterized as follows:

The first class of antisense oligonucleotides, also referred to herein as second generation of antisense oligonucleotides, comprises a total of 23 nucleotides comprising in 5' → 3' direction a stretch of seven 2'-O-methylribonucleotides, a stretch of nine 2'-deoxyribonucleotides, a stretch of six 2'-O-methylribonucleotides and a 3'-terminal 2'-deoxyribonucleotide. From the first group of seven 2'-O-methylribonucleotides the first four are phosphorothioate linked, whereas the subsequent four 2'-O-methylribonucleotides are phosphodiester linked. Also, there is a phosphodiester linkage between the last, i. e. the most 3'-terminal end of the 2'-O-methylribonucleotides and the first nucleotide of the stretch consisting of nine 2'-deoxyribonucleotides. All of the 2'-deoxyribonucleotides are phosphorothioate linked. A phosphorothioate linkage is also present between the last, i. e. the most 3'-terminal 2'-deoxynucleotide, and the first 2'-O-methylribonucleotide of the subsequent stretch consisting of six 2'-O-methylribonucleotides. From this group of six 2'-O-methylribonucleotides the first four of them, again in 5' → 3' direction, are phosphodiester linked, whereas the last three of them, corresponding to positions 20 to 22 are phosphorothioate linked. The last, i. e. terminal 3'-terminal 2'-deoxynucleotide is linked to the last, i. e. most 3'-terminal 2'-O-methylribonucleotide through a phosphorothioate linkage.

This first class may also be described by reference to the following schematic structure: RRRnnnnNNNNNNNNNnnnRRRN. Hereby, R indicates phosphorothioate linked 2'-O-methyl ribonucleotides (A, G, U, C); n stands for 2'-O-methyl ribonucleotides (A, G, U, C); N represents phosphorothioate linked deoxyribonucleotides (A, G, T, C).

The second class of particularly preferred antisense oligonucleotides, also referred to herein as third generation (of) antisense oligonucleotides or GeneBlocs, also comprises a total of 17 to 23 nucleotides with the following basic structure (in 5' → 3' direction).

At the 5'-terminal end there is an inverted abasic nucleotide which is a structure suitable to confer resistance against exonuclease activity and, e. g., described in WO 99/54459. This inverted abasic is linked to a stretch of five to seven 2'-O-methylribonucleotides which are phosphodiester linked. Following this stretch of five to seven 2'-O-methylribonucleotides there is a stretch of seven to nine 2'-deoxyribonucleotides all of which are phosphorothioate linked. The linkage between the last, i. e. the most 3'-terminal 2'-O-methylribonucleotide and the first 2'-deoxynucleotide of the 2'-deoxynucleotide comprising stretch occurs via a phosphodiester linkage. Adjacent to the stretch of seven to nine 2'-deoxynucleotides a stretch consistent of five to seven 2'-O-methylribonucleotides is connected. The last 2'-deoxynucleotide is linked to the first 2'-O-methylribonucleotide of the latter mentioned stretch consisting of five to seven 2'-O-methylribonucleotides occurs via a phosphorothioate linkage. The stretch of five to seven 2'-O-methylribonucleotides are phosphodiester linked. At the 3'-terminal end of the second stretch of five to seven 2'-O-methylribonucleotide another inverted abasic is attached.

This second class may also be described by reference to the following schematic structure: (GeneBlocs representing the 3rd generation of antisense oligonucleotides have also the following schematic structure:) cap-$(n_p)_x(N_s)_y(n_p)_z$-cap or cap-nnnnnnnNNNNNNNNNnnnnnnn-cap. Hereby, cap represents inverted deoxy abasics or similar modifications at both ends; n stands for 2'-O-methyl ribonucleotides (A, G, U, C); N represents phosphorothioate-linked deoxyribonucleotides (A, G, T, C); x represents an integer from 5 to 7; y represents an integer from 7 to 9; and z represents an integer from 5 to 7.

It is to be noted that the integers x, y and z may be chosen independently from each other although it is preferred that x and z are the same in a given antisense oligonucleotide. Accordingly, the following basic designs or structures of the antisense oligonucleotides of the third generation can be as follows: cap-$(n_p)_5(N_s)_7(n_p)_5$-cap, cap-$(n_p)_6(N_s)_7(n_p)_5$-cap, cap-$(n_p)_7(N_s)_7(n_p)_5$-cap, cap-$(n_p)_5(N_s)_8(n_p)_5$-cap, cap-$(n_p)_6(N_s)_8(n_p)_5$-cap, cap-$(n_p)_7(N_s)_8(n_p)_5$-cap, cap-$(n_p)_5(N_s)_9(n_p)_5$-cap, cap-$(n_p)_6(N_s)_9(n_p)_5$-cap, cap-$(n_p)_7(N_s)_9(n_p)_5$-cap, cap-$(n_p)_5(N_s)_7(n_p)_6$-cap, cap-$(n_p)_6(N_s)_7(n_p)_6$-cap, cap-$(n_p)_7(N_s)_7(n_p)_6$-cap, cap-$(n_p)_5(N_s)_8(n_p)_6$-cap, cap-$(n_p)_6(N_s)_8(n_p)_6$-cap, cap-$(n_p)_7(N_s)_8(n_p)_6$-cap, cap-$(n_p)_5(N_s)_9(n_p)_6$-cap, cap-$(n_p)_6(N_s)_9(n_p)_6$-cap, cap-$(n_p)_7(N_s)_9(n_p)_6$-cap, cap-$(n_p)_5(N_s)_7(n_p)_7$-cap, cap-$(n_p)_6(N_s)_7(n_p)_7$-cap, cap-$(n_p)_7(N_s)_7(n_p)_7$cap, cap-$(n_p)_5(N_s)_8(n_p)_7$-cap, cap-$(n_p)_6(N_s)_8(n_p)_7$-cap, cap-$(n_p)_7(N_s)_8(n_p)_7$-cap, cap-$(n_p)_5(N_s)_9(n_p)_7$-cap, cap-$(n_p)_6(N_s)_9(n_p)_7$-cap and cap-$(n_p)_7(N_s)_9(n_p)_7$-cap.

Ribozymes are catalytically active nucleic acids which preferably consist of RNA which basically comprises two moieties. The first moiety shows a catalytic activity whereas the second moiety is responsible for the specific interaction with the target nucleic acid, in the present case the nucleic acid coding for PpIX. Upon interaction between the target nucleic acid and the second moiety of the ribozyme, typically by hybridisation and Watson-Crick base pairing of essentially complementary stretches of bases on the two hybridising strands, the catalytically active moiety may become active which means that it catalyses, either intramolecularly or intermolecularly, the target nucleic acid in case the catalytic activity of the ribozyme is a phosphodiesterase activity. Subsequently, there may be a further degradation of the target nucleic acid which in the end results in the degradation of the target nucleic acid as well as the protein derived from the said target nucleic acid. Ribozymes, their use and design principles are known to the one skilled in the art, and, for example described in Doherty and Doudna (Ribozym structures and mechanism. Annu ref. Biophys. Biomolstruct. 2001 ; 30 :457-75) and Lewin and Hauswirth (Ribozyme Gene Therapy: Applications for molecular medicine. 2001 7: 221-8).

Use of the nucleic acid-based molecules of the invention will lead to better treatment of the disease progression by affording the possibility of combination therapies (e. g., multiple RNA interference mediating or triggering nucleic acid molecules targeted to different genes; nucleic acid molecules coupled with known small molecule modulators; or intermittent treatment with combinations of molecules, including different motifs and/or other chemical or biological molecules). The treatment of subjects with nucleic acid molecules according to the present invention can also include combinations of different types of nucleic acid molecules, such as enzymatic nucleic acid molecules (ribozymes), allozymes, antisense, 2,5-A oligoadenylate, decoys, and aptamers. This kind of nucleic acid molecules are known to the one skilled in the art. Additionally, a treatment combined with the use of the nucleic acid molecules according to the present invention, is photochemotherapy as, for example, described in US 6,710,066 the disclosure of which is incorporated herein by reference. A particularly preferred combined therapeutic approach is the use of the nucleic acid molecule according to the present invention together with ALA. Such combined use results in a significantly increased PpIX fluorescence which propels the treatment of neoplastic tissues and thus neoplastic diseases. This kind of photodynamic therapy will be conducted sevral hours after application of ALA and siRNA for Ferrochelatase. A dye laser then delivers light at a wavelength of 635 nm, e.g. through a 600-mm quartz fiber inserted down the biopsy channel of a flexible videoendoscope. siRNA can be applied locally or systemically, or by using a siRNA-producing vector.

In another aspect a nucleic acid molecule of the invention comprises one or more 5' and/or a 3'-cap structure, for example on only the first strand, the second strand, or both strands of the nucleic acid molecule according to the present invention.

By "cap structure" is meant chemical modifications, which have been incorporated at either terminus of the oligonucleotide (see, for example, Adamic et al., U.S. Pat. No. 5,998,203, incorporated by reference herein). These terminal modifications protect the nucleic acid molecule from exonuclease degradation, and may help in delivery and/or localization within a cell. The cap my be present at the 5'-terminus (5'-cap) or at the 3'-terminal (3'-cap) or may be present on both termini. In non-limiting examples, the 5'-cap is selected from the group consisting of glyceryl, inverted deoxy abasic residue (moiety); 4',5'-methylene nucleotide; 1-(beta-D-erytbrofuranosyl) nucleotide, 4'-thio nucleotide; carbocyclic nucleotide; 1,5-anhydrohexitol nucleotide; L-nucleotides; alpha-nucleotides; modified base nucleotide; phosphorodithioate linkage; *threo*-pentofuranosyl nucleotide; acyclic 3',4'-seco nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety.

In non-limiting examples, the 3'-cap is selected from the group consisting of glyceryl, inverted deoxy abasic residue (moiety), 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate; 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; *threo*-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'phosphoramidate; phosphorothioate and/or phosphorodithioate, bridging or non bridging methylphosphonate and 5'-mercapto moieties (for more details see Beaucage and Iyer, 1993, Tetrahedron 49, 1925; incorporated by reference herein).

By the term "non-nucleotide" preferably means any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine and therefore lacks a base at the 1'-position.

An "alkyl" group preferably refers to a saturated aliphatic hydrocarbon, including straight-chain, branched-chain, and cyclic alkyl groups. Preferably, the alkyl group has 1 to 12 carbons. More preferably, it is a lower alkyl of from 1 to 7 carbons, more preferably 1 to 4 carbons. The alkyl group can be substituted or unsubstituted. When substituted the substituted group(s) is preferably, hydroxyl, cyano, alkoxy, =O, =S, $NO_2$ or $N(CH_3)_2$, amino, or SH. The term also includes alkenyl groups that are unsaturated hydrocarbon groups containing at least one carbon-carbon double bond, including straight-chain, branched-chain, and cyclic groups. Preferably, the alkenyl group has 1 to 12 carbons. More preferably, it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, =O, =S, $NO_2$, halogen, $N(CH_3)2$, amino, or SH. The term "alkyl" also includes alkynyl groups that have an unsaturated hydrocarbon group containing at least one carbon-carbon triple bond, including straight-chain, branched-chain, and cyclic groups. Preferably, the alkynyl group has 1 to 12 carbons. More preferably, it is a lower alkynyl of from 1 to 7 carbons, more preferably 1 to 4 carbons. The alkynyl group ma be substituted or unsubstituted. When substituted the substituted group(s) is preferably, hydroxyl, cyano, alkoxy, =O, =S, $NO_2$ or $N(CH_3)2$, amino or SH.

Such alkyl groups can also include aryl, alkylaryl, carbocyclic aryl, heterocyclic aryl, amide and ester groups. An "aryl" group refers to an aromatic group that has at least one ring having a conjugated pi electron system and includes carbocyclic aryl, heterocyclic aryl and biaryl groups, all of which may be optionally substituted. The preferred substituent (s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above). Carbocyclic aryl groups are groups wherein the ring atoms on the aromatic ring are all carbon atoms. The carbon atoms are optionally substituted. Heterocyclic aryl groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the llike, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

By "nucleotide" as preferably used herein is as recognized in the art to include natural bases (standard), and modified bases well known in the art. Such bases are generally located at the 1' position of a nucleotide sugar moiety. Nucleotides generally comprise a base, sugar and a phosphate group. The nucleotides can be modified at the sugar, phosphate and/or base moiety, (also referred to interchangeably as nucleotide analogs, modified nucleotides, non-natural nucleotides, non-standard nucleotides and other; see, for example, Usman and McSwiggen, *supra;* Eckstein et al., International PCT Publication No. WO 92/07065; Usman et al., International PCT Publication No. WO 93/15187; Uhlman & Peyman, *supra,* all are hereby incorporated by reference herein). There are several examples of modified nucleic acid bases known in the art as summarized by Limbach et al., 1994, Nucleic Acids Res. 22, 2183. Some of the non-limiting examples of base modifications that can be introduced into nucleic acid molecules include, inosine, purine, pyridine-4-one, pyridine-2-one, phenyl, pseudouracil, 2, 4, 6-trimethoxy benzene, 3-methyl uracil, dihydrouridine, naphthyl, aminophenyl, 5-alkyleytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, and others (Burgin et al., 1996, Biochemistry, 35, 14090; Uhlman & Peyman, supra). By "modified bases" in this aspect is meant nucleotide bases other than adenine, guanine, cytosine and uracil at 1'position or their equivalents.

In one embodiment, the invention features modified nucleic acid molecules, with phosphate backbone modifications comprising one or more phosphorothioate, phosphorodithioate, methylphosphonate, phosphotriester, morpholino, amidate carbamate, carboxymethyl, acetatmidate, polyamide, sulfonate, sulfonamide, sufamate, formacetal, thioformacetal, and/or alkylsilyl, substitutions. For a review of oligonucleotide backbone modifications, see Hunziker and Leumann, 1995, Nucleic Acid Analogues: Synthesis and Properties, in Modem Synthetic Methods, VCH, 331-417, and Mesmaeker et al., 1994, Novel Backbone Replacements for Oligonucleotides, in Carbohydrate Modifications in Antisense Research, ACS, 24-39.

By "abasic" is preferably meant sugar moieties lacking a base or having other chemical groupos in place of a base at the 1' position, see for example Adamic et al., U.S. Pat. No. 5,998,203.

By "unmodified nucleoside" is preferably meant one of the bases adenine, cytosine, guanine, thymine, or uracil joined to the 1' carbon of β-ribo-furanose.

By "modified nucleoside" is preferably meant any nucleotide base which contains a modification in the chemical structure of an unmodified nucleotide base, sugar and/or phosphate. Non-limiting examples of modified nucleotides are shown by Formulae I-VII and/or other modifications described in UK patent 2 397 062.

In connection with 2'-modified nucleotides as described for the present invention, by "amino" is preferably meant 2'-$NH_2$ or 2'-O-$NH_2$, which can be modified or unmodified. Such modified groups are described, for example, in Eckstein et al., U.S. Pat. No. 5,672,695 and Matulic-Adamic et al., U.S. Pat. No. 6,248,878, which are both incorporated by reference in their entireties.

Various modifications to nucleic acid molecules according to the present invention can be made to enhance the utility of these molecules. Such modifications will enhance shelf-life, half-life in vitro, stability, and case of introduction of such oligonucleotides to the target site, e.g., to enhance penetration of cellular membranes, and confer the ability to recognize and bind to targeted cells.

The nucleic acid molecules according to the present invention can preferably be adapted for use as medicaments and diagnostics, alone or in combination with other therapies. For example, a nucleic acid molecule according to the present invention can comprise a delivery vehicle, including liposomes, for administration to a subject, carriers and diluents and their salts, and/or can be present in pharmaceutically acceptable formulations. Methods for the delivery of nuclecic acid molecules are described in Akhtar et al., 1992, Trends Cell Bio., 2, 139; Delivery Strategies for Antisense Oligonucleotde Therapeutics, ed. Akhtar, 1995, Maurer et al., 1999, Mol. Memb. Biol., 16, 129-140; Hofland and Huang, 1999, Handb. Exp. Pharmacol., 137, 165-192; and Lee et al., 2000, ACS Symp. Ser., 752, 184-192 all of which are incorporated herein by reference. Beigelman et al., U.S. Pat. No. 6,395,713 and Sullivan et al., PCT WO 94/02595 further describe the general methods for delivery of nucleic acid molecules. These protocols can be utilized for the delivery of virtually any nucleic acid molecule. Nucleic acid molecules can be administered to cells by a variety of methods known to those of skill in the art, including, but not limited to, encapsulation in liposomes, by iontophoresis, or by incorporation into other vehicles, such as hydrogels, cyclodextrins (see for example Gonzalez et al., 1999, Bioconjugate Chem., 10, 1068-1074), biodegradable nanocapsules, and bioadhesive microspheres, or by proteinaccous vectors (O'Hare and Normand, International PCT Publication No. WO 00/53722). Alternatively, the nucleic acid/vecicle combination is locally delivered by direct injection or by use of an infusion pump. Direct injection of the nucleic acid molecules of the invention, whether subcutaneous, intramuscular, or intradermal, can take place using standard needle and syringe methodologies, or by needle-free technologies such as those described in Conry et al., 1999, Clin. Cancer Res., 5, 2330-2337 and Barry et al., International PCT Publication No. WO 99/31262. The molecules of the instant invention can be used as pharmaceutical agents. Preferably, pharmaceutical agents prevent, modulate the occurrence, or treat (alleviate a symptom to some extent, preferably all of the symptoms) of a disease state in a subject.

Thus, there is provided a pharmaceutical composition comprising one or more nucleic acid(s) according to the present invention in an acceptable carrier, such as a stabilizer, buffer, and the like. The polynucleotides of the invention can be administered (e.g., RNA, DNA or protein) and introduced into a subject by any standard means, with or without stabilizers, buffers, and the like, to form a pharmaceutical composition. When it is desired to use a liposome delivery mechanism, standard protocols for formation of liposomes can be followed. The compositions of the present invention can also be formulated and used as tablets, capsules or elixirs for oral administration, suppositories for rectal administration, sterile solutions, suspensions for injectable administration, and the other compositions known in the art.

There are further provided pharmaceutically acceptable formulations of the nucleic acid molecules according to the present invention. These formulations include salts of the above compounds, e.g., acid addition salts, for example, salts of hydrochloric, hydrobromic, acetic acid, and benzene sulfonic acid.

A pharmacological composition or formulation refers to a composition or formulation in a form suitable for administration, e.g., systemic administration, into a cell or subject, including for example a human. Suitable forms, in part, depend upon the use or the route of entry, for example oral, transdermal, or by injection. Such forms should not prevent the composition or formulation from reaching a target cell (i.e., a cell to which the negatively charged nucleic acid is desirable for delivery). For example, pharmacological compositions injected into the blood stream should be soluble. Other factors are known in the art, and include considerations such as toxicity and forms that prevent the composition or formulation from exerting its effect.

By "systemic administration" is meant in vivo systemic absorption or accumulation of drugs in the blood stream followed by distribution throughout the entire body. Administration routes that lead to systemic absorption include, without limitation: intravenous, subcutaneous, intraperitoneal, inhalation, oral, intrapulmonary and intramuscular. Each of these administration routes exposes the siNA molecules of the invention to an accessible diseased tissue. The rate of entry of a drug into the circulation has been shown to be a function of molecular weight or size. The use of a liposome or other drug carrier comprising the nucleic acids according to the present invention can potentially localize the drug, for example, in certain tissue types, such as neoplastic tissue(s). A liposome formulation that can facilitate the association of drug with the surface of cells, such as lymphocytes and macrophages is also useful. This approach can provide enhanced delivery of the drug to target cells by taking advantage of the specificity of macrophage and lymphocyte immune recognition of abnormal cells, such as cells forming the neoplastic tissue.

By "pharmaceutically acceptable formulation" is meant, a composition or formulation that allows for the effective distribution of the nucleic acid molecules according to the present invention in the physical location most suitable for their desired activity. Nori-limiting examples f or agents suitable for formulation with the nucleic acid molecules according to the present invention include: P-glycoprotein inhibitors (such as Pluronic P85), which can enhance entry of drugs into the CNS (Jollict-Riant and Tillement, 1999, Fundam. Clin. Pharmacol., 13, 16-26); biodegradable polymers, such as poly (DL-lactide-coglycolide) microspheres for sustained release delivery after intracerebral implantation (Emerich, DF et al., 1999, Cell Transplant, 8, 47-58) (Alkermes, Inc. Cambridge, MA); and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999). Other non-limiting examples of delivery strategies for the nucleic acid molecules of the instant invention include material described in Boado et al., 1998, J. Pharm. Sci., 87, 1308-1315; Tyler et al., 1999, FEBS Lett., 421, 280-284; pardridge et al., 1995, PNAS USA., 92,5592-5596; Boado, 1995, Adv. Drug Delivery Rev., 15,73-107; Aldrian-Herrada et al., 1998, Nucleic Acids Res., 26, 4910-4916; and Tyler et al., 1999, PNAS USA., 96, 7053-7058.

There is also provided the use of the composition comprising surface-modified liposomes containing poly (ethylene glycol) lipids (PEG-modified, or long-circulating liposomes or stealth liposomes). These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al.,

Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995,42,24864-24780; Choi et al., Internaional PCT Publication No. WO 96/10391; Ansell et al., International PCT Publication No. WO 96/10390; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues suc has the liver and spleen.

There are moreover provided compositions prepared for storage of administration that include a pharmaceutically effective amount of the desired compounds in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A.R. Gennaro edit. 1985), hereby incorporated by reference herein. For example, preservatives, stabilizers, dyes and flavoring agent can be provided. These include sodium benzoate, sorbic acid and esters of p-hydroxybenzoic acid. In addition, antioxidants and suspending agents can be used.

A pharmaceutically effective dose is that dose required to prevent, inhibit the occurrence, or threat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors that those skilled in the medical arts will recognize. Generally, an amount between 0.1 mg/kg and 100 mg/kg body weight/day of active ingredients is administered dependent upon potency of the negatively charged polymer.

The nucleic acid molecules according to the present invention and formulations thereof can be administered orally, topically, parenterally, by inhalation or spray, or rectally in dosage unit formulations containing conventional non-toxic pharmaceutically acceptable carriers, adjuvants and/or vehicles. The term parenteral as used herein includes percutaneous, subcutaneous, intravascular (e.g., intravenous), intramuscular, or intrahecal injection or infusion techniques and the like. In addition, there is provided a pharmaceutical formulation comprising a nucleic acid molecule of the invention and a pharmaceutically acceptable carrier. One or more nucleic acid molecules according to the present invention can be present in association with one or more non-tocxic pharmaceutically acceptable carriers and/or diluents and/or adjuvants, and if desired other active ingredients. The pharmaceutical compositions containing nucleic acid molecules according to the present invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be, for example, inert diluents; such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in a mixture with excipients suitable for the manufacture of aqueous suspensions. Such excipients are suspending agents, for example sodium carboxymethylcellulose, methylcellulose, hydropropylmethylcellulose, sodium alginate, polyvinylpyrrolidone, gum tragacanth and gum acacia; dispersing or wetting agents can be a naturally-occuring phosphatide, for example lecithin, or condensation products of an alkylene oxide with fatty acids, for example polyoxyethylene stearate, or condensation products of ethylene oxide with long chain aliphatic alcohols, for example heptadecaethyleneoxyoctanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavouring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavouring agents can be added to provide palatable oral preparations. These compositions can be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavouring and coloring agents, can also be present.

Pharmaceutical compositions of the invention can also be in the form of oil-in-water emulsions. The oily phase can be a vegetable oil or a mineral oil or mixture of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxycthylene sorbitan monooleate. The emulsions can also contain sweetening and flavouring agents.

Syrups and elixirs can be formulated with sweetening agents, for example glycerol, propylene glycol, sorbitol, glucose or sucrose. Such formulations can also contain a demulcent, a preservative and flavouring and coloring agents. The pharmaceutical compositions can be in the from of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono- or diglycerides. In addition, fatty acids such as oleic acid find use in the preparation of injectables.

The nucleic acid molecules of the invention can also be administered in the form of suppositories, e. g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Nucleic acid molecules of the invention can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels for the medicament and pharmaceutical composition, respectively, can be determined by those skilled in the art by routine experimentation.

It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

For administration of the medicament according to the present invention to non-human animals such as dogs, cats, mice, rats, guinea pigs, monkeys, sheep, goats, cattle, and horses, the composition can preferably also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules of the present invention can also be administered to a subject in combination with other therapeutic compounds to increase the overall therapeutic effect. The use of multiple compounds to treat an indication can increase the beneficial effects while reducing the presence of side effects.

In one embodiment, there are provided compositions suitable for administering the nucleic acid molecules according to the present invention to specific cell types, whereby such compositions typically incorporate one or several of the following principles and molecules, respectively. For example, the asialoglycoprotein receptor (ASGPr) (Wu and Wu, 1987, J. Biol. Chem. 262, 4429-4432) is unique to hepatocytes and binds branched galactose-terminal glycoproteins, such as asialoorosomucoid (ASOR). In another example, the folate receptor is overexpressed in many cancer cells. Binding of such glycoproteins, synthetic glycoconjugates, or folates to the receptor takes place with an affinity that strongly depends on the degree of branching of the oligonsaccharide chain, for example, tria-tennary structures are bound with greater affinity than biatenarry or monoatennary chains (Baenziger and Fiete, 1980, Cell, 22, 611-620; Connolly et al., 1982, J. Biol. Chem., 257, 939-945). Lee and Lee, 1987. Glycoconjugate J., 4, 317-328, obtained this high specificity through the use of N-acetyl-D-galactosamine as the carbohydrate moiety, which has higher affinity for the receptor, compared to galactose. This "clustering effect" has also been described for the binding and uptake of mannosyl-terminating glycoproteins or glycoconjugates (Ponpipom et al., 1981, J. Med. Chem., 24, 1388-1395). The use of galactose, galactosamine, or folate based conjugates to transport exogenous compounds across cell membranes can provide a targeted delivery approach to, for example, the treatment of liver disease, cancers of the liver, or other cancers. The use of bioconjugates can also provide a reduction in the required dose of therapeutic compounds required for treatment. Furthermore, therapeutic bioavailability, pharmacodynamics, and pharmacokinetic parameters can be modulated through the use of nucleic acid bioconjugates of the invention. Non-limiting examples of such bioconjugates are described in Vargeese et al., USSN 10/201,394, filed August 13, 2001; and Matulic-Adamic et al., USSN 60/362,016, filed March 6, 2002.

The nucleic acid molecules, in their various embodiments, according to the present invention, the vector, cell, medicament, composition and in particular pharmaceutical composition containing the same, tissue and animal, respectively, according to the present invention containing such a nucleic acid molecule can be used in both for a therapeutic as well as in the diagnostic and research field.

As the nucleic acid molecule according to the present invention is suitable to increase the intracellular concentration of the fluorescent PpIX, an effective labeling of the thus treated cells can be obtained. The labeling of such cells can be either transient or permanent, depending on the mode of administration and the particular construct used for the introduction and expression, respectively, of the nucleic acid molecule according to the present invention as is known to the one skilled in the art.

Preferred research fields where in particular the cell according to the present invention is useful, is the field of immunology and oncology, in vivo geno-typing and pheno-typing of transgenic animals, migration of cells as well as the study of adhesion phenomena, whereby the latter two aspects can also be used in a clinical setting, such as diagnosis, staging and follow-up treatment in connection with neoplastic diseases. The cells treated with the nucleic acid molecules according to the present invention can be detected making use of the fluorescence characteristics of PpIX which shows a high fluorescence in the red spectrum, whereby with excitation at 410 nm, emission occurs at 630 nm, and with excitation at 630 nm, emission occurs at 690 nm. Preferably the cell, tissue and organism, respectively, are watched through a fluorescence microscope or any other device which allows the visualizing of the emission wavelength and thus to spot the respective cell, tissue and organism, respectively. A particularly preferred characteristic of this kind of use is that the thus labeled cells are not destroyed and the disadvantages inherent to the exogenous administered dye ligand complex of the prior art, are avoided.

A further use of the nucleic acid molecules, the vector, the cell, the composition and the organism, respectively, according to the invention is for diagnosis, whereby the diagnosis is based on the afore-described fluorescence of PpIX.

From the medical point of view the recognition of neoplastic tissues based on the detection of increased PpIX-formation is of particular interest in the following diseases: cancers of the upper aerodigestive tract, distal esophagus, colorectum, urogenital tract: specific risk factors for the development of these tumour entities are known, which may serve for the identifications of risk groups. These groups may profit from screening investigations, if sufficiently sensitive and specific procedures were available. Screening in such risk groups could include drug application followed by subsequent endoscopic assessment. Screening may be directed towards (non-exclusive list): detection of preneoplastic and early neoplastic squamous cell lesions of the oropharynx, the tracheobronchial tree and the esophagus in long term smokers, detection of preneoplastic and early neoplastic adenomatous lesions in the distal esophagus associated with chronic acid reflux (Barrett esophagus), early neoplastic adenomatous lesions in the colon and rectum in association with known preneoplastic conditions like ulcerative colitis, or preneoplastic genetical disorders (FAP: Familial adenomatous polyposis, HNPCC: hereditary non-polyposis colon cancer), early transitional cell neoplasms in the urinary bladder, early cervix neoplasms in gynecology.

In a further embodiment the diagnostic method of the present invention can be used for the detection of the various diseases which are disclosed herein as being treatable by the means and methods according to the present invention and thus for the identification of the patients which can be treated in accordance with the present invention. In a further embodiment the diagnostic method is used for a follop-up of this kind of patients and more particularly of the patients treated in accordance with the present invention.

In connection with both the methods for the treatment and prevention and for the diagnosis of the diseases disclosed herein, the active agents, i.e. the nucleic acid molecules in accordance with the present invention such as siRNA molecules, antisense-molecules and ribozymes, are preferably administered locally, preferably locally into the tumors and the other tissues afflicted, respectively, and/or the cells forming or being part of such tumor and tissue, respectively. Alternatively, the vectors coding for such nucleic acid molecules in accordance with the present invention are expressed in such cells by tumor-specific and tissue-specific promoters, respectively. Tumor-specific promoters are preferably those which control genes which in turn are activated in such tumors. Respective promoters are known to the ones skilled in the art and comprise among others the VEGF promoter. An alternative way for a specific delivery of the active agent according to the present invention such as the nucleic acid molecule, is the delivery thereof by means of carriers. Such carrier may contain the nucleic acid molecule as such or a vector such as a plasmid coding therefore. Preferably the carrier provides means for the specific delivery. Means for specific delivery of such carriers are known to the ones skilled in the art. In a preferred embodiment the carrier comprises one or several antibodies, whereby the specificity of the delivery is provided by the antibody specifically interacting with a marker of the cells and tissue, respectively, which in turn are involved in the disease. More preferably, the antibody is directed against a tumor marker and the tumor marker is specific for tumor cells to be treated in accordance with the present invention.

The nucleic acid molecule, the vector, the cell and the composition, preferably the pharmaceutical composition and medicament, respectively, according to the present invention is, preferably and without wishing to be bound by any theory, effective based on the fluorescence of PpIX. More specifically, the fluorescence, upon suitable excitation by light and by high-energy radiation such as radioactive irradation, triggers photodynamic processes such as, but not limited to, the formation of singlet oxygen and oxygen radicals which are responsible for the destruction of the cells having such increased intracellular content of PpIX. More specifically, a particularly preferred procedure is such that the nucleic acid molecule according to the present invention is administered to the target cell, whereupon the content of PpIX is increased. The PpIX is activated by means of a laser. For the purpose of activation in a preferred embodiment optical fibers may be used for transmitting the energy required for triggering the photodynamic processes.

Particularly preferred si RNA molecules are as follows:

(1) 5’ gauucaagagcaguaccgc 3’ (second strand and second stretch, respectively)

3 ’cuaaguucucgucauggcg 5’ (first strand and first stretch, respectively)

(2) 5’gaauauccucuugguuccg 3’ (second strand and second stretch, respectively

3’cuuauaggagaaccaaggc 5’ (first strand and first stretch, respectively)

(3) 5’guacaguguucaugauacg 3’ (second strand and second stretch, respectively

3’ caugucacaaguacuaugc 5’ (first strand and first stretch, respectively)

The present invention is further illustrated by the following figures and examples from which further features, embodiments and advantages may be taken.

Fig. 1 shows a diagram indicating the result of a quantitative RT-PCR for ferrochelatase mRNA-expression in different parts of the gastrointestinal tract;

Fig. 2 shows a diagram indicating PpIX accumulation in different cell lines treated with ferrochelatase specific siRNA molecules.

## Example 1: Materials and Methods

If not indicated to the contrary, the following materials and methods were used in connection with the present invention and the subsequent examples.

### Materials & Chemicals

Taqman 7000 SDS Cycler and all real-time PCR chemicals were - unless otherwise stated - from Applied Biosystems (Weiterstadt, Germany). Agarose was obtained from BioWhittaker Molecular Applications (Rockland ME USA), Trizol LS reagent, Guanidine thiocyanate-phenol-chloroform, ethidium-bromide, Dulbecco’s Modified Eagle’s Medium, fetal calf serum, phosphate buffered saline, antibiotics and glutamine were obtained from GIBCO, Life Tech. (Eggenstein, Germany). RiboGreen RNA quantification reagent was obtained from Molecular Probes (Leiden, Netherlands), BSA Fraction 5 and glutamine from PAA (Cölbe, Germany). M MLV Reverse Transcriptase and RNasin, RNaseH, dNTP were purchased from ProMega (Mannheim, Germany), siRNA, Transfection messenger reagent, RNeasy RNAextraction Kit, QIAshredder Kit, One-Step RT PCR Kit, and QiaEx-II gel extraction Kit from Qiagen (Hilden, Germany). Sucrose was obtained from Serva (Heidelberg, Germany), Protoporphyrin IX disodium salt and Protoporphyrin IX zinc (II), Triton X-100, Linear polyacrylamide (GenElute LPA), BSA and Mayer’s Hematoxylin Solution from Sigma-Aldrich (Munich, Germany). Phenol/Chloroform and all other chemicals were purchased from Roth (Karlsruhe, Germany).

### Patients and tissue samples

Surgical specimens were obtained from the tumor bank of the Robert-Roessle-Clinic. Patient specimens were collected and used in accordance with Institutional Review Board approved protocols and guidelines. Tissue was snap-frozen in liquid nitrogen. Specimens were classified and characterized according to the WHO/UICC guidelines.

Samples derived from colonic tissue of three patient groups were examined by microarray analysis (Table 1): colonic tissue of healthy individuals that underwent coloscopy (HEALTHY), colonic cancer tissue of patients with a low risk of tumour-dependent death (LOW) and colonic cancer tissue of patients with a high-risk of tumour-dependent death (HIGH). Colonic tissue of healthy individuals that underwent coloscopy was non-inflamed tissue, free of adenomas or carcinomas, according to histopathological analysis. Most of these patients were suspected of having cancer due to blood within the stool. Besides patient survival, the low-risk group (LOW) was defined by a low T stage, no lymph node (N=0) or distant metastases (M=0), no signs of tumour recurrence, of metachronous metastases or other carcinomas. The high-risk group (HIGH) was defined by a low T stage, neither lymph node (N=0) nor distant metastases at the time of surgery (M=0). In three of the five cases of HIGH, metachronous metastases have been formed. All specimens have been resected without any residual tumour left. The mean survival time of the low-risk group is 2507 days (about 6.8 years); mean age at the time of surgery was 67 years. The mean survival time of the high-risk group is 1365 days (about 3,7 years); mean age at surgery was 71 years. For subsequent quantitative RT PCR studies, another panel of colorectal carcinoma cases was used, with well-documented histological characteristics and a comprehensive clinical record.

***Table 1: Histopathological data of samples used for the microarray experiments***

**Tables 1a,b. Histopathological data of the cases under study.** Carcinomas were classified and characterized according to the WHO/UICC system. Abbreviations: Case, patient number; Age, age in years at time of surgery; T, invasiveness; N, metastasis to regional lymph nodes; M, synchronous formation of distant metastases; G, grading; L, lymphatic infiltration; V, venous infiltration; Alive, Yes, if patient has survived more than 4 years after surgery; Survival, survival in days; Mpost, metachronous formation of distant metastases.

**Table 1a. Histopathological data of the low-risk group (LOW)**

| Case | Age | T | N | M | G | L | V | Alive | Survival | Mpost |
|---|---|---|---|---|---|---|---|---|---|---|
| 1 | 63 | 2 | 0 | 0 | 2 | 0 | 0 | Yes | 2669 | No |
| 2 | 73 | 1 | 0 | 0 | 2 | 0 | 0 | Yes | 3025 | No |
| 3 | 75 | 2 | 0 | 0 | 2 | 0 | 0 | Yes | 2309 | No |
| 4 | 58 | 1 | 0 | 0 | 2 | 0 | 0 | Yes | 1775 | No |
| 5 | 67 | 3 | 0 | 0 | 1 | 0 | 0 | Yes | 2758 | No |

**Table 1b. Histopathological data of the high-risk group (HIGH)**

| Case | Age | T | N | M | G | L | V | Alive | Survival | Mpost |
|---|---|---|---|---|---|---|---|---|---|---|
| 6 | 73 | 1 | 0 | 0 | 1 | 0 | 0 | No | 878 | Yes |
| 7 | 68 | 3 | 0 | 0 | 3 | 0 | 0 | No | 582 | No |
| 8 | 71 | 2 | 0 | 0 | 2 | 0 | 0 | No | 907 | No |
| 9 | 66 | 1 | 0 | 0 | 2 | 0 | 0 | Yes | 2534 | Yes |
| 10 | 68 | 2 | 0 | 0 | 1 | 0 | 0 | No | 1877 | Yes |

The distribution of stages in this group was as follows:

For quantitative RT-PCR studies, a panel of 18 cases of esophageal carcinomas, 20 cases of gastric carcinomas, 15 cases of colonic carcinomas, 15 cases of rectal carcinomas, and their non malignant counterparts were examined. For each patient a comprehensive histopathological and clinical record is available.

**RNA-extraction for microarray analysis.**

Total RNA was isolated using the Trizol LS reagent under addition of linear polyacrylamide (GenElute LPA). RNA quality and amount were checked using the Bio-Analyzer technology (Agilent). Equivalent amounts of RNA from five tissues of each type (HEALTHY, LOW, or HIGH) were pooled. About 2 μg total RNA (10 μl) was heated to 70°C for 10 min in the presence of 1 μl (500 ng/μl) of an anchored oligo(dT) primer containing the T7 RNA polymerase promoter T7-primer 1 [5' GGC CAG TGA ATT GTA ATA CGA CTC ACT ATA GGG AGG CGG(T)24], cooled on ice and reverse transcribed with 400 U Superscript II (SSII), 1 μl dNTP mix (10 mM), 2 μl DTT (0.1 M) and 4 μl 5X First strand cDNA buffer for 1 h at 42°C. Second strand synthesis was carried out by adding 30 μl 5X second-strand buffer (100 mM Tris-HCl, 225 mM KCl, 7.5 mM MgCl2, 0.125 mg/ml BSA, 7.5 mM DTT), 3 μl dNTP (10mM), 4 μl E. coli DNA polymerase I (10 U/ μl), 1 μl RNaseH (2U/ μl) and 91 μl DEPC-H2O and incubation for 2 h at16°C. The resulting cDNA was treated with 2 μl T4 DNA Polymerase (10 U/ μl) for 10 min at 16°C, extracted with Phenol/Chloroform and Phase Lock Gels, washed twice with 70 % ethanol and resuspended in DEPC-water. T7 RNA-polymerase based biotinylation was carried out using the T7-Transcription kit as recommended by the manufacturer. No further amplification of the sample material was carried out. Fragmentation was carried out as recommended. Pooled RNA populations were accessed in duplicate or quadruplicate, i.e. several independent samples from the same biological condition have been prepared. The healthy stage was profiled in quadruplicate; primary carcinomas of the low-risk or high-risk group in duplicate.

**Oligonucleotide Microarray Analysis.**

Expression spectra were accessed by usage of the complete U95 GeneChip Set (Affymetrix, Santa Clara, CA). The five chips of the U95 series enable to monitor simultaneously the transcription of about 12,000 known genes (U95A) and about 48,000 ESTs (U95B-E). Initial acquisition of expression information was performed by Affymetrix Microarray Suite 5.0 software. Primary scanning data was processed with the program GeneSpring (Silicon Genetics, San Carlos, CA). Normalization was carried out as follows: For each array, the median of all signals was used as a calibrator for the entire chip, in the way that each gene's signal was divided by the median value. As control for correct background subtraction, the bottom tenth percentile was used. Sequence annotations were updated via Internet. For GeneChip analysis, gene lists were created for each of the five different chip types U95A, B, C, D, E (20 lists, totally): First, for each chip type sequences up- or downregulated at least fourfold in cancer patients (high- and low-risk individuals combined) compared to healthy subjects were extracted into two different lists. Subsequently, for each of the chips transcripts, whose abundances were in- or decreased by at least a factor of four between the high- and low-risk groups, were put in another list. In addition, the following criteria have to be fulfilled for list membership: signal variations across repetitive samples were not allowed to exceed a factor of 0.4, and flags (a measure of how reliable a certain expression value can be regarded as) had to display the designator "present" at least once or twice in the up-regulated condition, depending on if two or four samples were available, respectively. Subsequently, Mann-Whitney U test in conjunction with Benjamini and Hochberg false discovery rate multiple testing corrections was performed in order to see if list sequences displayed a differential expression that is statistically significant.

**Tissue Preparation and RNA Extraction for RT-PCR.**

Cryo-sections from colorectal and normal tissue specimens were transferred into RLT-Buffer containing β-mercaptoethanol and frozen at -80° C. Only specimens containing more than 60% epithelial cells, no Peyer's patches and no necrotic areas were further processed. RNA was isolated using QIAshredder and RNeasy Kits. Integrity of isolated mRNA was checked by β-*Actin* One-Step RT PCR, and RNA concentration was determined photometrically. About 3 μg of RNA were used for cDNA synthesis. In the presence of 4 μM random hexamer primer RNA was incubated for 10 min at 70° C, then the mixture was hold on ice for another 10 min. After addition of M-MLV-Buffer, 8 U/μL M-MLV Reverse Transcriptase, 0.8 U/μL RNasin, 0.1 μg/μL BSA and 1.25 mM dNTP and 1 h incubation at 37° C, twice the volume of absolute ethanol was added. Following 30 min at - 40° C, samples were spun down in a centrifuge at 15,000 g at 4° C for 20 min and washed once with 70% ethanol. Finally, cDNA was reconstituted in 50 μL DEPC water and frozen away at - 20° C.

**Quantitative Real-Time Taqman PCR.**

For quantitative analysis of gene expression always 100 ng of cDNA were used per well and all reactions were carried out in triplicate. In all cases Assays-on-Demand (ABI) were employed, for which primer and probe sequences are kept in secret by the manufacturer. After mixing with the appropriate volume of TaqMan Universal PCR Master Mix, quantitative real-time PCR was run in a MicroAmp Optical 96-Well Reaction Plate using the ABI 7000 Sequence Detection System. Thermal cycle conditions were as follows: 95° C for 10 min, initially, then 40 cycles of 95° C for 15 s and 60° C for 1 min.

In order to analyze expression data according to the $\Delta\Delta C_t$ method (11), $C_t$ values were exported from the ABI Prism 7000 SDS Software into Microsoft Excel (Seattle, WA). $C_t$ values of a cDNA stock from the cell line LS 174T were used as calibrator. Thereby gene expression in a sample under investigation is reported as a multiple of cell culture expression. This is achieved by employing the equation

wherein . $\Delta C_t$ stands for the difference between $C_t$ values of gene of interest and housekeeper β-*Actin.*

$$\text{Relative Amount} = 2^{-(\Delta Ct_{Sample} - \Delta Ct_{Calibrator})},$$

**PpIX-determination**

PpIX fluorescence was excited with the help of an optical parametric oscillator (OPO, GWU-Lasertechnik, Erftstadt, Germany), pumped by the third harmonic ($\lambda$ = 355 nm, $E_{pulse}$ = 100 mJ) of a Q-switched Nd:YAG laser (GCR-230, Spectra-Physics Inc., USA). The OPO was tuned to provide laser radiation at $\lambda$ = 505 nm to excite PpIX-fluorescence. The energy and the duration of output pulses amounted typically to 5 mJ and 3 ns, respectively. The laser beam was coupled into a 600 μm hard cladding silica fiber used for illumination of tissue samples. The fluorescence light was collected by the same fiber and a dichroic beam splitter served to separate reflected laser light from fluorescence. About 5 % of the remaining fluorescence intensity was separated by a quartz plate and coupled into a hard clad silica fiber, about 5 m long, whereas the main fluorescence intensity (90 %) was focused into a second fiber, 1 m long. In this way fluorescence in the first channel is delayed with respect to the second channel by about 20 ns allowing to record prompt and delayed fluorescence intensity simultaneously. For this purpose the ends of both fibers were attached one above the other to the entrance slit of an imaging polychromator equipped with an intensified CCD-camera (Model ICCD-576, Princeton Instruments Inc, Trenton, USA.). The intensifier was gated by an electrical pulse (-180 V) of about 10 ns duration derived from a high voltage (HV) pulse generator synchronized with the laser pulse. The HV pulse generator was triggered by a pulse provided by the power supply of the Nd:YAG laser and appropriately delayed by means of a digital delay generator. In this way immediate and delayed fluorescence intensities were recorded. Furthermore, two long pass filters were used ($\lambda_{50\%}$ = 550 nm) to cut off scattered excitation light from emitted fluorescence light.

For quantification we calculated the normalized fluorescence intensity of the main PpIXfluorescence band at $\lambda$=633 nm. The factor 18 corresponds to the ratio of the transmitted and reflected fluorescence intensity by the quartz plate.

**Ferrochelatase (FC) activity.**

FC activity was determined as described by (6) with minor modifications. Briefly, serial cryostat sections were examined by a pathologist. Only tissue pieces containing predominantly carcinoma tissue were analyzed. About 100 mg of tumor tissue was powdered in liquid N2 using a tissue-homogenizer (Braun, Germany) and was took in 2 ml H2O. After 10 min. of centrifugation at 20000 x g, 50 μl of the supernatant was added to 100 μl buffer A, consisting of 25 mM Tris/HCl, 0.1 % Triton X-100, 1.75 mM mM palmitic acid, pH 8.2. Protein in the supernatant was determined using the BioRad protein assay. Fifty μl of a 250 μM protoporphyrin solution in 0.01 N KOH was added, and the reaction was started by addition of 50 μl 200 μM zinc acetate. After 60 min. at 37°C, the reaction was stopped by addition of 1 ml dimethylsulfoxide:methanol (30:70). After centrifugation at 20000xg for 10 min, 100 μl supernatant was injected on a Shimadzu HPLC with a reversed-phase RP18 column with acetone/methanol/water/formic acid (560:240:200:2), 1 ml /min as the mobile phase. Zinc-protoporphyrin was detected by a Merck fluorimeter excitation 405 nm, emission 580 nm. One unit of activity was defined as the amount of enzyme catalysing the formation of 1 μmol product/min. The activity of the enzyme is given in nU/ mg protein in the supernatant.

**Example 2: Ferrochelatase (FC) mRNA-expression in gastrointestinal tumor biopsies**

Ferrochelatase mRNA expression in microdissected specimens of gastrointestinal tissue biopsies was determined by quantitative single-step multiplex RT-PCR in relation to that of β-Actin and compared with mRNA expression of the colorectal cell line SW480. In a second series of experiments, FC expression in colonic and rectal carcinomas was determined in relation to another "housekeeping gene", namely hypoxanthine phosphoribosyltransferase (HPRT) by quantitative RT-PCR as well (data not shown). The data of this second experiment are in good agreement with results obtained by using β-actin. FC expression in colorectal mucosa of healthy individuals was much higher than in mucosa of tumor patients (Table 5) FC mRNA-expression was significantly reduced in gastric (P<0.001), colonic (P=0.005) and rectal (P=0.031) carcinomas compared to corresponding normal mucosa (Figure 1, Wilcoxon-test of matched pairs). FC-levels were particularly low in esophageal tissues. Although not significant, FC expression of esophageal carcinomas was also much lower than that of corresponding mucosa.

*Table 5. mRNA-Expression of Ferrochelatase (FC) in colorectal carcinomas*

| | N | FC | Stdev |
|---|---|---|---|
| Normal | 30 | 3,57 | 2,90 |
| Carcinoma | 30 | 2,08 | 1,51 |

Ferrochelatase mRNA expression was determined by quantitative RT-PCR. FC mRNA expression was significantly higher in normal mucosa compared to carcinoma tissues. (P=0.005, Wilcoxon-test). Computational analyis was done using the Delta-Delta Ct -method in comparison to β-actin expression.

Abbreviations: N, number of cases; FC, relative amount of FC-expression; Stdev, Standard deviation of relative amount.

**Example 3: FC mRNA-expression of cell lines correlates with their potential to accumulate PpIX after ALA-stimulation**

In an in vitro model consisting of several colorectal carcinoma lines FC mRNA-expression and FC enzyme activity were determined independently. PpIX-accumulation was induced by exogenous ALA-stimulation with 0.1 mM ALA for 210 min and determined by time-delayed fluorescence measurements as described above.

For determination of mRNA-expression quantitative RT-PCR (Taqman) was used. Computational analyis of expression data was done using the Delta-Delta Ct -method with reference to β-actin expression. Enzyme activity was determined using Zn-protoprophyrin as substrate and measured using HPLC with fluorescent detection. Protoporphyrin was determined by time-delayed fluorescence of frozen cell pellets (see Materials& Methods).

The results are depicted in the following table.

| Cell line | mRNA-expression (relative amount) | enzyme activity (μU/mg) | PpIX (nmol/$10^6$ cells) |
|---|---|---|---|
| **HT29-P92** | 0.526 | 8.917 | 16.963 |
| **SW480** | 0.551 | 10.917 | 27.944 |
| **HT29-P37** | 0.696 | 12.333 | 19.153 |
| **MDA-MB-435** | 0.921 | 18.333 | 0.732 |
| **LS174T** | 1.000 | 23.542 | 1.761 |

On the one hand Ferrochelatase enzyme activity showed a strong correlation with mRNA expression ($r^2$=0.95). On the other hand, cell lines with high FC enzyme activity displayed low PpIX-fluorescence ($r^2$=0.71).

**Example 4: Inhibition of FC-expression by siRNA-treatment**

The expression of FC was inhibited by siRNA-treatment of the colorectal carcinoma cell line LS 147T, which expressed the highest amount of FC on the mRNA and enzyme activity level. Accordingly, this cell line showed only a small PpIX-fluorescence in previous experiments. We used siRNA-sequences directed towards three different regions of the coding region of human ferrochelatase (FC) for silencing, starting from basepair 381, 1011 and 1980 in a concentration of 50 nM. In addition a 50 nM mixture composed of all 3 sequences was used, and cells were treated with siRNA directed towards a sialyltransferase enzyme ("other") not related to heme biosynthesis. Most effective in FC downregulation was the siRNA-sequence FC-1011, which blocked 67 % of the FC expression found in mock-treated cells. The results are depicted in the following table.

| LS147T | FC | β-Actin | dCt | ddCt | rel. amount | % inhibition |
|---|---|---|---|---|---|---|
| **mock** | 23.5 | 17.1 | 6.38 | 0.00 | 1.0000 | 0 % |
| **381** | 24.6 | 16.7 | 7.91 | 1.52 | 0.3481 | 65 % |
| **1011** | 24.7 | 16.7 | 7.96 | 1.58 | 0.3347 | 67 % |
| **1980** | 24.5 | 17.0 | 7.47 | 1.08 | 0.4719 | 53 % |

(continued)

| LS147T | FC | β-Actin | dCt | ddCt | rel. amount | % inhibition |
|---|---|---|---|---|---|---|
| **mix** | 26.0 | 18.6 | 7.42 | 1.03 | 0.4882 | 51 % |
| **other** | 28.38 | 20.85 | 7.53 | 0.01 | 0.9931 | 1 % |

After treatment with siRNA FC-1011 a strong PpIX-fluorescence was observed (Fig. 2). Thus, siRNA-treatment led to a drastically increased PpIX-fluorescence. No PpIX was found in cells treated with a siRNA towards the sialyltransferase.

**Example 5: PpIx accumulation after combined FC siRNA-treatment and ALA-stimulation**

Inhibition of FC-expression was achieved by treatment of cells with siRNA directed towards the FC sequence starting from basepair 1011 in a concentration of 50 nm as described above. Cells of the colorectal carcinoma line LS 147T , which in native condition showed almost no PpIX-fluorescence were treated for 5 days with FC siRNA 1011, thereafter ALA was added in a concentration of 0.1 μM ALA for 210 min (Table 7). PpIX fluorescence was increased more than 50-fold by combined treatment with siRNA FC-1011 or FC 381 and ALA compared to ALA-treatment alone.

Table 7 PpIX accumulation after combined FC siRNA-treatment and ALA-stimulation

| | **siRNA alone** | **siRNA + ALA** |
|---|---|---|
| **LS147T** | **PpIX** nM/$10^5$ cells | **PpIX** nM/$10^5$ cells |
| **Mock** | 0.0 | 2.7 |
| | | |
| **381** | 2.2 | 28.3 |
| **1011** | 15.9 | 111.9 |
| **1980** | 11.8 | 50.4 |
| **mix** | 12.5 | 78.0 |

Inhibition of FC-expression was achieved by treatment of cells with siRNA directed towards the FC sequence starting from basepair 1011 in a concentration of 50 nm. Cells of the colorectal carcinoma line LS147T, which in native condition showed almost no PpIX-fluorescence were treated for 5 days with FC siRNA 1011, thereafter ALA was added in a concentration of 0.1 μM ALA for 210 min. PpIX fluorescence was increased more than 50-fold by combined treatment with siRNA FC-1011 or FC-381 and ALA compared to ALA-treatment alone. Abbreviations: siRNA, Ferrochelatase specific siRNA; ALA, aminolevulinic acid; mock, mock-transfected LS 147T cells; 381,1011,1980,mix, siRNA-sequences;

The features of the present invention disclosed in the specification, the sequence listing, the claims and/or the drawings may both separately and in any combination thereof be material for realizing the invention in various forms thereof.

## Claims

1. A nucleic acid molecule comprising a double-stranded structure, whereby the double-stranded structure comprises a first strand and a second strand, whereby the first strand comprises a first stretch of contiguous nucleotides and the first stretch is at least partially complementary to a target nucleic acid, and whereby the second strand comprises a second stretch of contiguous nucleotides and whereby said second stretch is at least partially complementary to the first stretch, whereby the first stretch comprises a nucleic acid sequence which is complementary to a nucleotide sequence starting from position 383, 1013 or 1982 of the nucleic acid according to SEQ.ID.No. 1.

2. The nucleic acid according to claim 1, whereby the first stretch and/or the second stretch comprises from 18 to 29 consecutive nucleotides, preferably 19 to 25 consecutive nucleotides and more preferably 19 to 23 consecutive nucleotides.

3. The nucleic acid according to claim 1 and 2, whereby the first strand consists of the first stretch and/or the second strand consists of the second stretch.

4. The nucleic acid according to claim 3, whereby

- the first stretch consists of a nucleotide sequence according to SEQ.ID.No.2 and/or whereby the second stretch consists of a nucleotide sequence according to SEQ.ID.No.3,
- the first stretch consists of a nucleotide sequence according to SEQ.ID.No.4 and/or whereby the second stretch consists of a nucleotide sequence according to SEQ.ID.No.5,
- the first stretch consists of a nucleotide sequence according to SEQ.ID.No.6 and/or whereby the second stretch consists of a nucleotide sequence according to SEQ.m.No.7.

5. The nucleic acid according to any of claims 1 to 4, whereby the first stretch comprises a plurality of group of modified nucleotides having a modification at the 2' position, whereby within the stretch each group of modified nucleotides is flanked on one or both sides by a flanking group of nucleotides, whereby the flanking nucleotides forming the flanking group of nucleotides is either an unmodified nucleotide or a nucleotide having a modification different from the modification of the modified nucleotides.

6. The nucleic acid according to any of claims 1 to 5, whereby first stretch and/or the second stretch comprises a pattern of groups of modified nucleotides and/or a pattern of flanking groups of nucleotides.

7. The nucleic acid according to any of claims 1 to 6, preferably claims 3 and 4, whereby first stretch and/or the second stretch comprise at the 3' end a dinucleotide, whereby such dinucleotide is preferably TT.

8. The nucleic acid according to claim 7, whereby the length of the first stretch and/or of the second stretch consists of either 19 or 21 nucleotides.

9. The nucleic acid according to any of claims 1 to 4, preferably 3 and 4, whereby the first and/or the second stretch comprise an overhang of 1 to 5 nucleotides at the 3' end.

10. The nucleic acid according to claim 9, whereby the length of the double-stranded structure is from about 16 to 24 nucleotide pairs, preferably 20 to 22 nucleotide pairs.

11. The nucleic acid according to any of claims 1 to 10, whereby the first strand and the second strand are covalently linked to each other, preferably the 3' end of the first strand is covalently linked to the 5' end of the second strand.

12. A vector, preferably an expression vector, comprising or coding for a nucleic acid according to any of claims 1 to 11.

**13.** A composition, preferably a pharmaceutical composition, comprising a nucleic acid according to any of claims 1 to 11 or a vector according to claim 12.

**14.** The composition according to claim 13, whereby the composition is a pharmaceutical composition for the treatment of a disease, whereby the disease is preferably selected from the group comprising neoplastic diseases, inflammatory and other systemic diseases.

**15.** Use of a nucleic acid according to any of claims 1 to 11 for the manufacture of a medicament, whereby such medicament is preferably used together with photodynamic treatment, radiotherapy, hyperthermia or chemotherapy.

**Figure 1**

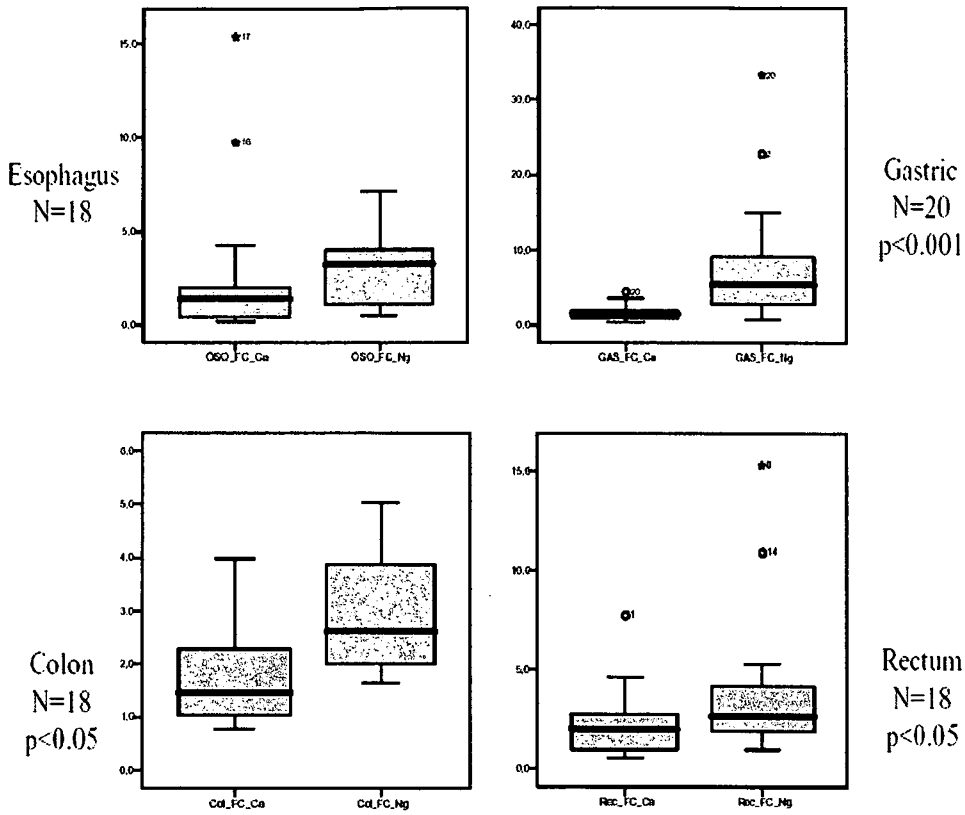
Esophagus
N=18
Gastric
N=20
p<0.001
Colon
N=18
p<0.05
Rectum
N=18
p<0.05
OSO_FC_Ca
OSO_FC_Ng
GAS_FC_Ca
GAS_FC_Ng
Col_FC_Ca
Col_FC_Ng
Rec_FC_Ca
Rec_FC_Ng

**Figure 2**

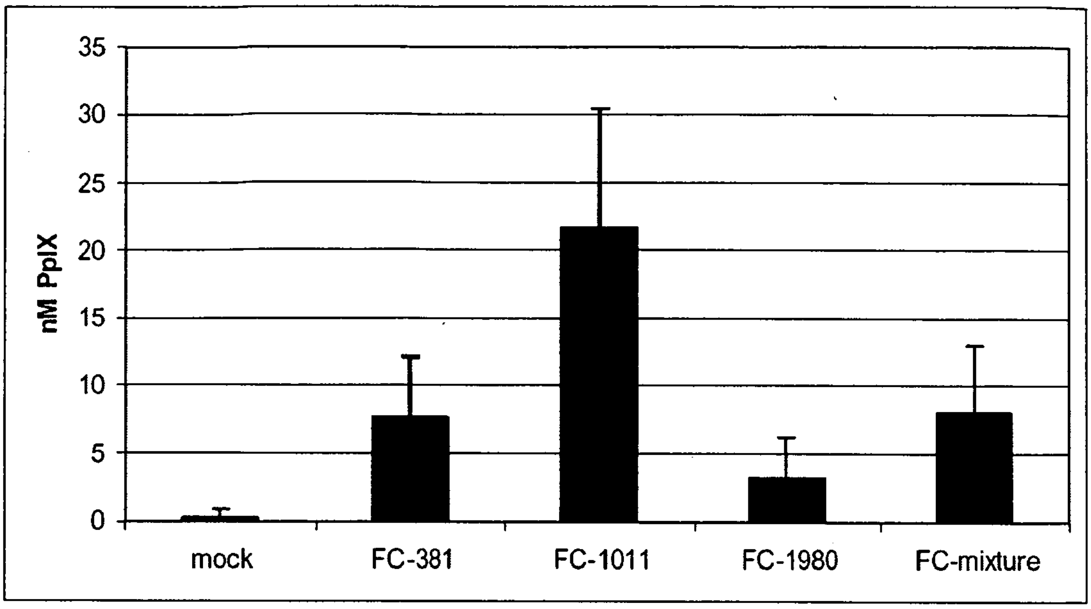
35
30
25
20
15
10
5
0
nM PpIX
mock
FC-381
FC-1011
FC-1980
FC-mixture

European Patent Office

# EUROPEAN SEARCH REPORT

Application Number
EP 06 01 8993

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BHASIN GAYATRI ET AL: "Protoporphyrin-IX accumulation and cutaneous tumor regression in mice using a ferrochelatase inhibitor." CANCER LETTERS, vol. 187, no. 1-2, 10 December 2002 (2002-12-10), pages 9-16, XP002418776 ISSN: 0304-3835 * the whole document * ----- | 1-15 | INV. C12N15/11 A61K31/713 ADD. A61P35/00 |
| A | EP 1 389 637 A (ATUGEN AG [DE]) 18 February 2004 (2004-02-18) * the whole document * ----- | 1-15 | |
| A | WO 2004/015107 A2 (ATUGEN AG [DE]; GIESE KLAUS [DE]; KAUFMANN JOERG [DE]; KLIPPEL-GIESE A) 19 February 2004 (2004-02-19) * the whole document * ----- | 1-15 | TECHNICAL FIELDS SEARCHED (IPC) |
| D,A | HUA Z ET AL: "EFFECTIVENESS OF DELTA-AMINOLEVULINIC ACID-INDUCED PROTOPORPHYRIN AS A PHOTOSENSITIZER FOR PHOTODYNAMIC THERAPY IN VIVO" CANCER RESEARCH, vol. 55, no. 8, 15 April 1995 (1995-04-15), pages 1723-1731, XP002912997 ISSN: 0008-5472 ----- | | C12N C07K A61K |

The present search report has been drawn up for all claims

| Place of search | Date of completion of the search | Examiner |
|---|---|---|
| The Hague | 7 February 2007 | Andres, Serge |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 01 8993

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2007

| Patent document cited in search report | Publication date | Patent family member(s) | Publication date |
|---|---|---|---|
| EP 1389637 A | 18-02-2004 | ZA 200500459 A | 18-07-2005 |
| WO 2004015107 A2 | 19-02-2004 | AT 350473 T | 15-01-2007 |
| | | AU 2003260370 A1 | 25-02-2004 |
| | | BR 0313202 A | 21-06-2005 |
| | | CA 2494930 A1 | 19-02-2004 |
| | | CN 1675359 A | 28-09-2005 |
| | | JP 2006500014 T | 05-01-2006 |
| | | KR 20050035877 A | 19-04-2005 |
| | | MX PA05001355 A | 30-09-2005 |
| | | US 2004180351 A1 | 16-09-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004015107 A
- EP 0586520 B1
- EP 0618925 B1
- WO O175164 A
- WO 0244321 A
- WO 03070910 A
- GB 2397062 A
- WO O170949 A
- WO 9954459 A, Thompson
- US 6001311 A, Brennan
- WO 9323569 A, Draper
- WO 9207065 A, Eckstein
- WO 9103162 A, Usman
- US 5334711 A, Sproat
- US 6300074 B, Gold
- WO 9315187 A, Usman
- WO 9726270 A
- US 5716824 A
- US 5627053 A, Usman
- WO 9813526 A, Woolf
- US 082404 P, Thompson
- WO 0066604 A, Wengel
- WO 9914226 A
- US 5854038 A, Sullenger and Cech
- US 5849902 A
- US 5989912 A
- US 6710066 B
- US 5998203 A, Adamic
- US 5672695 A, Eckstein
- US 6248878 B, Matulic-Adamic
- US 6395713 B, Beigelman
- WO 9402595 A, Sullivan
- WO 0053722 A, O'Hare and Normand
- WO 9931262 A, Barry
- WO 9610391 A, Choi
- WO 9610390 A, Ansell
- WO 9610392 A, Holland
- US 20139401 A, Vargeese
- US 36201602 P, Matulic-Adamic


### Non-patent literature cited in the description


- **BARR H. et al.** *Lancet,* 1996, vol. 348, 584-585
- **KOENIG, F. et al.** *Science,* 2001, vol. 292, 1401-1403
- **GOSSNER, L et al.** *Cancer,* 1999, vol. 86, 1921-1928
- **VAN HELEGERSBERG, R et al.** *Gastroenterology,* 1992, vol. 103, 647-651
- **HUA, Z.** *Cancer Res,* 1995, vol. 55, 1723-1731
- **HINNEN, P et al.** *J. Cancer,* 1998, vol. 78, 679-682
- **TAKETANI, S et al.** *Eur. J. Biochem.,* 2000, vol. 267, 4685-4692
- **DAILEY, H. A.** *Biochem. J.,* 1984, vol. 223, 441-445
- **STEPINAC T ; FELLEY C ; JORNOD P ; LANGE N ; GABRECHT T ; FONTOLLIET C ; GROSJEAN,P ; VANMELLE G ; VAN DEN BERGH H ; MONNIER P.** Endoscopic fluorescence detection of intraepithelial neoplasia in Barrett's esophagus after oral administration of aminolevulinic acid. *Endoscopy,* 2003, vol. 35, 663-668
- **MAYINGER B ; NEIDHARDT S ; REH H ; MARTUS P ; HAHN E.** Fluorescence induced with 5-aminolevulinic acid for the endoscopic detection and follow-up of esophageal lesions. *Gastrointest Endosc,* 2001, vol. 54, 572-578
- **HINNEN P ; DE ROOIJ F ; VAN VELTHUYSEN M ; EDIXHOVEN A ; VAN HILLEGERSBERG R ; TILANUS H ; WILSON J ; SIERSEMA P.** Biochemical basis of 5-aminolaevulinic acid-induced protoporphyrin IX accumulation: a study in patients with (pre)malignant lesions of the oesophagus. *Br J Cancer,* 1998, vol. 78, 679-682
- **KLINTEBERG C ; ENEJDER A ; WANG I ; ANDERSSON-ENGELS S ; SVANBERG S ; SVANBERG K.** Kinetic fluorescence studies of 5-aminolevulinic acid induced protoporphyrin IX accumulation in basal cell carcinomas. *J Photochem Photobiol B,* 1999, vol. 49, 120-128
- **OLIVO M ; LAU W ; MANIVASAGER V ; HOON T ; CHRISTOPHER C.** Fluorescence confocal microscopy and image analysis of bladder cancer using 5-aminolevulinic acid. *Int J Oncol,* 2003, vol. 22, 523-528

- **KRIEGMAIR M ; ZAAK D ; KNUECHEL R ; BAUMGARTNER R ; HOFSTETTER A.** 5-Aminolevulinic acid-induced fluorescence endoscopy for the detection of lower urinary tract tumors. *Urol Int,* 1999, vol. 63, 27-31
- **KRIEG,R.C ; FICKWEILER,S ; WOLFBEIS,O.S ; KNUECHEL,R.** Cell-type specific protoporphyrin IX metabolism in human bladder cancer in vitro. *Photochem.Photobiol.,* 2000, vol. 72, 226-233
- **PENG Q ; MOAN J ; WARLOE T ; NESLAND J ; RIMINGTON C.** Distribution and photosensitizing efficiency of porphyrins induced by application of exogenous 5-aminolevulinic acid in mice bearing mammary carcinoma. *Int J Cancer,* 1992, vol. 52, 433-443
- **PONKA P.** Cell biology of heme. *Am J Med Sci,* 1999, vol. 318, 241-256
- **SAUTER E ; EHYA H ; KLEIN-SZANTO A ; WAGNER-MANN C ; MACGIBBON B.** Fiberoptic ductoscopy findings in women with and without spontaneous nipple discharge. *Cancer,* 2005, vol. 103, 914-921
- **NAVONE N ; POLO C ; FRISARDI A ; ANDRADE N ; BATLLE A.** Heme biosynthesis in human breast cancer - mimetic 'in vitro' studies ad soe heme enzymic activity levels. *Int J Biochem,* 1990, vol. 22, 1407-1411
- **LADNER D ; STEINER R ; ALLEMANN J ; HALLER U ; WALT H.** Photodynamic diagnosis of breast tumours after oral application of aminolevulinic acid. *Br J Cancer,* 2001, vol. 84, 33-37
- **FREI K ; BONEL H ; FRICK H ; WALT H ; STEINER R.** Photodynamic detection of diseased axillary sentinel lymph node after oral application of aminolevulinic acid in patients with breast cancer. *Br J Cancer,* 2004, vol. 90, 805-809
- **DORWARD,A.M ; FANCHER,K.S ; DUFFY,T.M ; BEAMER,W.G ; WALT,H.** Early neoplastic and metastatic mammary tumours of transgenic mice detected by 5-aminolevulinic acid-stimulated protoporphyrin IX accumulation. *Br.J.Cancer,* 2005, vol. 93, 1137-1143
- **BOGAARDS A ; AALDERS M ; ZEYL C ; DE BLOK S ; DANNECKER C ; HILLEMANNS P ; TEPP H ; STERENBORG H.** Localization and staging of cervical intraepithelial neoplasia using double ratio fluorescence imaging. *J Biomed Opt,* 2002, vol. 7, 215-220
- **MOESTA K ; EBERT B ; HANDKE T ; NOLTE D ; NOWAK C ; HAENSCH W ; PANDREY R ; DOUGHERTY T ; RINNEBERG H ; SCHLAG P.** Protoporphyrin IX occurs naturally in colorectal cancers and their metastases. *Cancer Res,* 2001, vol. 61 (9), 91-999
- **KRIEG R ; MESSMANN H ; RAUCH J ; SEEGER S ; KNUECHEL R.** Metabolic characterization of tumor cell-specific protoporphyrin IX accumulation after exposure to 5-aminolevulinic acid in human colonic cells. *Photochem Photobiol,* 2002, vol. 76, 518-525
- **ENDLICHER,E ; GELBMANN,C.M ; KNUCHEL,R ; FURST,A ; SZEIMIES,R.M ; GOLDER,S.K ; SCHOLMERICH,J ; LOTTNER,C ; MESSMANN,H.** Hexaminolevulinate-induced fluorescence endoscopy in patients with rectal adenoma and cancer: a pilot study. *Gastrointest.Endosc.,* 2004, vol. 60, 449-454
- Specific intensity imaging for glioblastoma and neural cell cultures with 5-aminolevulinic acid-derived protoporphyrin IX. *J.Neurooncol.,* 2005, vol. 71, 107-111
- **CHEN J ; MAK N ; CHEUNG N ; LEUNG R ; PENG Q.** Endogenous production of protoporphyrin IX induced by 5-aminolevulinic acid in leukemia cells. *Acta Pharmacol Sin,* 2001, vol. 22, 163-168
- **FURRE,I.E ; SHAHZIDI,S ; LUKSIENE,Z ; MOLLER,M.T ; BORGEN,E ; MORGAN,J. ; TKACZ-STACHOWSKA,K ; NESLAND,J.M ; PENG,Q.** Targeting PBR by hexaminolevulinate-mediated photodynamic therapy induces apoptosis through translocation of apoptosis-inducing factor in human leukemia cells. *Cancer Res.,* 2005, vol. 65, 11051-11060
- **REBEIZ,N ; ARKINS,S ; KELLEY,K.W ; DURACK, G ; REBEIZ,C.A.** Modulator of heme biosynthesis induces apoptosis in leukemia cells. *J.Clin.Laser Med.Surg,* vol. 19, 59-67
- **BHASIN,G ; KAUSAR,H ; ATHAR,M.** Protoporphyrin-IX accumulation and cutaneous tumor regression in mice using a ferrochelatase inhibitor. *Cancer Lett.,* 2002, vol. 187, 9-16
- **STUMMER W ; STOCKER S ; WAGNER S ; STEPP H ; FRITSCH C ; GOETZ C ; GOETZ A ; KIEFMANN R ; REULEN H.** Intraoperative detection of malignant gliomas by 5-aminolevulinic acid-induced porphyrin fluorescence. *Neurosurgery,* 1998, vol. 42, 518-525
- **WU,S.M ; REN,Q.G ; ZHOU,M.O ; PENG,Q ; CHEN,J.Y.** Protoporphyrin IX production and its photodynamic effects on glioma cells, neuroblastoma cells and normal cerebellar granule cells in vitro with 5-aminolevulinic acid and its hexylester. *Cancer Lett.,* 2003, vol. 200, 123-131
- **BETZ C ; STEPP H ; JANDA P ; ARBOGAST S ; GREVERS G ; BAUMGARTNER R ; LEUNIG A.** A comparative study of normal inspection, autofluorescence and 5-ALA-induced PPIX fluorescence for oral cancer diagnosis. *Int J Cancer,* 2002, vol. 97, 245-252

- **LOENING M ; DIDDENS H ; KU¨PKER W ; DIEDRICH K ; HU¨TTMANN G.** Laparoscopic fluorescence detection of ovarian carcinoma metastases using 5-aminolevulinic acid-induced protoporphyrin IX. *Cancer,* 2004, vol. 100, 1650-1656
- **OTAKE M ; NISHIWAKI M ; KOBAYASHI Y ; BABA S ; KOHNO E ; KAWASAKI T ; FUJISE Y ; NAKAMURA H.** Selective accumulation of ALA-induced PpIX and photodynamic effect in chemically induced heptocellular carcinoma. *Br J Cancer,* 2003, vol. 89, 730-736
- **SMITS,T. ; ROBLES,C.A ; VAN ERP,P.E ; VAN DE KERKHOF,P.C ; GERRITSEN,M.J.** Correlation between macroscopic fluorescence and protoporphyrin IX content in psoriasis and actinic keratosis following application of aminolevulinic acid. *J.Invest Dermatol,* 2005, vol. 125, 833-839
- **SMITS,T ; ROBLES,C.A ; VAN ERP,P.E ; VAN DE KERKHOF,P.C ; GERRITSEN,M.J.** Correlation between macroscopic fluorescence and protoporphyrin IX content in psoriasis and actinic keratosis following application of aminolevulinic acid. *J.Invest Dermatol.,* 2005, vol. 125, 833-839
- **HE,D ; BEHAR,S ; NOMURA,N ; SASSA,S ; TAKETANI,S ; LIM,H.W.** The effect of porphyrin and radiation on ferrochelatase and 5-aminolevulinic acid synthase in epidermal cells. *Photodermatol.Photoimmunol.Photomed,* 1995, vol. 11, 25-30
- **DEVESA,I ; FERRANDIZ,M.L ; GUILLEN,I ; CERDA,J.M ; ALCARAZ,M.J.** Potential role of heme oxygenase-1 in the progression of rat adjuvant arthritis. *Lab Invest,* 2005, vol. 85, 34-44
- **LIU,Y.L ; ANG,S.O ; WEIGENT,D.A ; PRCHAL, J.T ; BLOOMER,J.R.** Regulation of ferrochelatase gene expression by hypoxia. *Life Sci.,* 2004, vol. 75, 2035-2043
- **FIRE et al.** *Nature,* 1998, vol. 391, 806
- **FIRE et al.** *Trends Genet.,* 1999, vol. 15, 358
- **STERNBERGER et al.** *Antisense. Nucl. Ac. Drug Dev.,* 2002
- **NYKANEN et al.** *Cell,* 2001, vol. 107, 309-21
- **ELAYADI ; COREY.** *Curr Opin Investig Drugs.,* 2001, vol. 2 (4), 558-61
- **ORUM ; WENGEL.** *Curr Opin Mol Ther.,* 2001, vol. 3 (3), 239-43
- **GOOD et al.** *Gene Ther,* 1997, vol. 4, 45-54
- **CARUTHERS et al.** *Methods in Enzymology,* 1992, vol. 211, 3-19
- **WINCOTT et al.** *Nucleic Acids Res.,* 1995, vol. 23, 2677-2684
- **WINCOTT et al.** *Methods Mol. Bio.,* 1997, vol. 74, 59
- **BRENNAN et al.** *Biotechnol Bioeng.,* 1998, vol. 61, 33-45
- **USMAN et al.** *J Am. Chem. Soc.,* 1987, vol. 109, 7845
- **SCARINGE et al.** *Nucleic Acids Res.,* 1990, vol. 18, 5433
- **MOORE et al.** *Science,* 1992, vol. 256, 9923
- **SHABAROVA et al.** *Nucleic Acids Research,* 1991, vol. 19, 4247
- **BELLON et al.** *Nucleosides & Nucleotides,* 1997, vol. 16, 951
- **BELLON et al.** *Bioconjugate Chem.,* 1997, vol. 8, 204
- **USMAN ; CEDERGREN.** *TIBS,* 1992, vol. 17, 34
- **USMAN et al.** *Nucleic Acids Symp. Ser.,* 1994, vol. 31, 163
- **PERRAULT et al.** *Nature,* 1990, vol. 344, 565
- **PIEKEN et al.** *Science,* 1991, vol. 253, 314
- **USMAN ; CEDERGREN.** *Trends in Biochem. Sci.,* 1992, vol. 17, 334
- **BURGIN et al.** *Biochemistry,* 1996, vol. 35, 14090
- **PERRAULT et al.** *Nature,* 1990, vol. 344, 565-568
- **PIEKEN et al.** *Science,* 1991, vol. 253, 314-317
- **USMAN ; CEDERGREN.** *Trends in Biochem. Sci,* 1992, vol. 17, 334-339
- **BEIGELMAN et al.** *J. Biol. Chem.,* 1995, vol. 270, 25702
- **KARPEISKY et al.** *Tetrahedron Lett.,* 1998, vol. 39, 1131
- **EAMSHAW ; GAIT.** *Biopolymers (Nucleic Acid Sciences,* 1998, vol. 48, 39-55
- **VERMA ; ECKSTEIN.** *Annu. Rev. Biochem.,* 1998, vol. 67, 99-134
- **BURLINA et al.** *Bioorg. Med. Chem,* 1997, vol. 5, 1999-2010
- **WINCOTT et al.** *Nucleic Acids Res.,* 1995, vol. 23, 2677
- **CARUTHERS et al.** *Methods in Enzymology,* vol. 211, 3-19
- **LIN ; MATTEUCCI.** *J. Am. Chem. Soc.,* 1998, vol. 120, 8531-8532
- **DOHERTY ; DOUDNA.** Ribozym structures and mechanism. *Annu ref. Biophys. Biomolstruct.,* 2001, vol. 30, 457-75
- **LEWIN ; HAUSWIRTH.** *Ribozyme Gene Therapy: Applications for molecular medicine,* 2001, vol. 7, 221-8
- **BEAUCAGE ; IYER.** *Tetrahedron,* 1993, vol. 49, 1925
- **LIMBACH et al.** *Nucleic Acids Res.,* 1994, vol. 22, 2183
- Nucleic Acid Analogues: Synthesis and Properties. **HUNZIKER ; LEUMANN.** Modem Synthetic Methods. VCH, 1995, 331-417
- **MESMAEKER et al.** Novel Backbone Replacements for Oligonucleotides. *Carbohydrate Modifications in Antisense Research,* 1994, 24-39
- **AKHTAR et al.** *Trends Cell Bio,* 1992, vol. 2, 139

- Delivery Strategies for Antisense Oligonucleotde Therapeutics. 1995
- **MAURER et al.** *Mol. Memb. Biol,* 1999, vol. 16, 129-140
- **HOFLAND ; HUANG.** *Handb. Exp. Pharmacol,* 1999, vol. 137, 165-192
- **LEE et al.** *ACS Symp. Ser,* 2000, vol. 752, 184-192
- **GONZALEZ et al.** *Bioconjugate Chem.,* 1999, vol. 10, 1068-1074
- **CONRY et al.** *Clin. Cancer Res.,* 1999, vol. 5, 2330-2337
- **JOLLICT-RIANT ; TILLEMENT.** *Fundam. Clin. Pharmacol,* 1999, vol. 13, 16-26
- **EMERICH, DF et al.** *Cell Transplant,* 1999, vol. 8, 47-58
- *Prog Neuropsychopharmacol Biol Psychiatry,* 1999, vol. 23, 941-949
- **BOADO et al.** *J. Pharm. Sci.,* 1998, vol. 87, 1308-1315
- **TYLER et al.** *FEBS Lett.,* 1999, vol. 421, 280-284
- **PARDRIDGE et al.** *PNAS USA,* 1995, vol. 92, 5592-5596
- **BOADO.** *Adv. Drug Delivery Rev,* 1995, vol. 15, 73-107
- **ALDRIAN-HERRADA et al.** *Nucleic Acids Res.,* 1998, vol. 26, 4910-4916
- **TYLER et al.** *PNAS USA,* 1999, vol. 96, 7053-7058
- **LASIC et al.** *Chem. Rev.,* 1995, vol. 95, 2601-2627
- **ISHIWATA et al.** *Chem. Pharm. Bull.,* 1995, vol. 43, 1005-1011
- **LASIC et al.** *Science,* 1995, vol. 267, 1275-1276
- **OKU et al.** *Biochim. Biophys. Acta,* 1995, vol. 1238, 86-90
- **LIU et al.** *J. Biol. Chem.,* 1995, vol. 42, 24864-24780
- Remington's Pharmaceutical Sciences. Mack Publishing Co, 1985
- **WU ; WU.** *J. Biol. Chem.,* 1987, vol. 262, 4429-4432
- **BAENZIGER ; FIETE.** *Cell,* 1980, vol. 22, 611-620
- **CONNOLLY et al.** *J. Biol. Chem.,* 1982, vol. 257, 939-945
- **LEE ; LEE.** *Glycoconjugate J.,* 1987, vol. 4, 317-328
- **PONPIPOM et al.** *J. Med. Chem.,* 1981, vol. 24, 1388-1395

SEQUENCE LISTING

<110> Max-Delbrück-Centrum für molekulare Medizin

<120> Means for increasing intracelllular levels of protoporphyrin IX and use thereof

<130> M 10056 EP

<160> 7

<170> PatentIn version 3.1

<210> 1

<211> 2443

<212> RNA

<213> Homo sapiens

<220>

<221> misc_feature

<223> mRNA of ferrochelatase

<400> 1
gggcgggucg ggccgaggcu gcccaggcaa ugcguucacu cggcgcaaac auggcugcgg 60

cccugcgcgc cgcgggcguc cugcuccgcg auccgcuggc auccagcagc uggaggggucu 120

gucagccaug gagguggaag ucaggugcag cugcagcggc cgucaccaca gaaacagccc 180

agcaugccca gggugcaaaa ccucaaguuc aaccgcagaa gaggaagccg aaaacuggaa 240

uauuaaugcu aaacauggga ggcccugaaa cucuuggaga uguucacgac uuccuucuga 300
gacucuucuu ggaccaagac cucaugacac uuccuauuca gaauaagcug gcaccauuca 360
ucgccaaacg ccgaaccccc aagauucaag agcaguaccg caggauugga ggcggauccc 420
ccaucaagau auggacuucc aagcagggag agggcauggu gaagcugcug gaugaauugu 480
cccccaacac agccccucac aaauacuaua uuggauuucg guacguccau ccuuuaacag 540
aagaagcaau ugaagagaug gagagagaug gccuagaaag ggcuauugcu uucacacagu 600
auccacagua cagcugcucc accacaggca gcagcuuaaa ugccauuuac agauacuaua 660
aucaaguggg acggaagccc acgaugaagu ggagcacuau ugacaggugg cccacacauc 720
accuccucau ccagugcuuu gcagaucaua uucuaaagga acuggaccau uuuccacuug 780
agaagagaag cgaggugguc auucuguuuu cugcucacuc acugccgaug ucugugguca 840
acagaggcga cccauauccu caggagguaa gcgccacugu ccaaaaaguc auggaaaggc 900
uggaguacug caacccccuac cgacuggugu ggcaauccaa gguugguccg augcccuggu 960
ugggucccuca aacagacgaa ucuaucaaag ggcuuuguga gagggggagg aagaauaucc 1020
ucuugguucc gauagcauuu accagugacc auauugaaac gcuguaugag cuggacaucg 1080
aguacucuca aguuuuagcc aaggagugug gaguugaaaa caucagaaga gcugagucuc 1140
uuaauggaaa uccauuguuc ucuaaggccc uggccgacuu ggugcauuca cacauccagu 1200
caaacgagcu guguuccaag cagcugaccc ugagcugucc gcucuguguc aauccugucu 1260
gcagggagac uaaauccuuc uucaccagcc agcagcugug acccccgccg guggaccccg 1320
uggcguuagg caaaugccca accuccagau accuccgaug uggagagggu guuauuuaga 1380
gaucaaggaa ggaagucauc cuuccuugau auauauacag ccuuugggua caaauugugu 1440
gguuucuuga ggauuggacu cuugauggau uucuauuuuu auauaacuau acaguaagca 1500
uuuguauuuu cucucucuag guauaaguua cuaguuugga auguccauca ggaccuuuaa 1560
uaaaugaggc uaaaaauuug ucuuaugaga cacaccuauu uaagcacaga uuuuggcuuu 1620
auugcccaaa acccucccga aaggguacgg agaguccccu cugugggcug gcagugugaa 1680
ugagaucugu uuagucucgu gcauauaguu gcuguuuuuu aaaugaacac aguugaguau 1740
uugaagugaa uuugaaaaag aaauguuacu uaaucuuucc cuaagcccau ggguuacaga 1800

augcuaggga ggcaauuugg uuaccugcaa uggcugcuuu ugccagcgag gccaccauuc 1860

auuggucauc uugguauuug ugcugugaau cucacuuucc ucaauguaaa aaggaaucaa 1920

guauggauuu cagaggugcu cuuagauucc ccauacaccc aaggguaaua aacguguaca 1980

aguacagugu ucaugauacg ugccuuggug ggaguccgug gugccacagg gaaggggcuc 2040

ccacugcuuc uggucuccag ggacagugcu gcuggaaagg cuagugauga gcuucacccu 2100

ggagcuccuc ccgggaccuu gcaagccucu ccauccagca ucuucucuau cuuaguugaa 2160

ugccuucuuu cugaacauuu guuuuaagaa uuauuuuaua aagucaacaa uacuuugcuu 2220

gaauucuuuc uuaauuuacg auuuuuuauu auaaaaaagu auagugauac aaugggacau 2280

gugaagaaua cagaaaagua accacuuuaa ugcaauaacu guuaucauaa uauuguauuu 2340

cgugguaguc cuugccugua gauauuuuua augccauuua augccauugu caccuuggau 2400

uuaugaguga aaaguguuuc uaaaaauaua gaaauaaugu cag 2443

<210> 2

<211> 19

<212> RNA

<213> Artificial

<220>

<221> misc_feature

<223> synthetic

<400> 2
gcgguacugc ucuugaauc 19

<210> 3

<211> 19

<212> RNA

<213> Artificial

<220>

<221> misc_feature

<223> synthetic

<400> 3
gauucaagag caguaccgc 19

<210> 4

<211> 19

<212> RNA

<213> Artificial

<220>

<221> misc_feature

<223> synthetic

<400> 4
cggaaccaag aggauauuc 19

<210> 5

<211> 19

<212> RNA

<213> Artificial

<220>

<221> misc_feature

<223> synthetic

<400> 5
gaauauccuc uugguuccg 19

<210> 6

<211> 19

<212> RNA

<213> Artificial

<220>

<221> misc_feature

<223> synthetic

<400> 6
cguaucauga acacuguac 19

<210> 7

<211> 19

<212> RNA

<213> Artificial

<220>

<221> misc_feature

<223> synthetic

<400> 7
guacaguguu caugauacg 19